# EUROPEAN PATENT APPLICATION

(11) **EP 3 058 953 A1**
(43) Date of publication of application: **24.08.2016**
(21) Application number: 16155784.8
(22) Date of filing: 07.07.2010
(51) Int. Cl.: A61K 39/00, A61K 9/127

(54) **LIPIDIC COMPOSITIONS FOR INDUCTION OF IMMUNE TOLERANCE**

(30) Priority: 07.07.2009 US 223521 P
(62) Divisional of application: 10797776.1
(71) Applicant: The Research Foundation Of State University Of New York, Amherst, NY 14228-2567 (US)
(72) Inventor: BALU-IYER, Sathy V., Amherst, NY 14228 (US); GAITONDE, Puneet Rajeev, Amherst, NY 14226 (US); BANKERT, Richard, Eden, NY 14057 (US)
(74) Representative: Appleyard Lees IP LLP

(57) **Abstract**

This invention provides a method for inducing immune tolerance toward an antigen comprising the antigen in lipidic particles or lipidic compositions. The lipidic particles are made up of phosphatidylserine and phosphatidylcholine, or phosphatidylinositol and phosphatidylcholine. The lipidic compositions comprise the antigen and O-phospho-L-serine. Administration of these composition results in inducing immune tolerance to the antigen.

## Description

### Field of the Invention

This invention relates to compositions and methods for inducing immune tolerance. More particularly, this invention provides lipidic compositions or particles which are useful for inducing immune tolerance.

### Description of Related Art

Advances in protein engineering have led to the development of proteins as therapeutic agents, but the safety and efficacy of protein therapeutics are often compromised by their immunogenicity. Formation of antibodies following administration of a protein therapeutic could have a profound impact on its pharmacology and efficacy. The antibodies can abrogate protein activity and/or alter their pharmacokinetic properties. Factors that can influence the immunogenicity of proteins include aggregation and frequency of administration.

Clearance mechanisms of protein drugs include proteolytic enzymes in the blood or interstitial fluids, and the internalization of protein by hepatic cells. In addition, protein drugs interact with cells of the immune system. Following protein uptake and processing by antigen presenting cells (APC), APC - T-cell interaction, and subsequent cytokine release shape the immune response.

Immunogenicity of proteins or peptides is also relevant to situations where development of immune tolerance is required (such as in allergic reactions). Accordingly, there is a need for methods to alleviate the immunogenicity of proteins or peptides and additionally, in the case of protein therapeutics, to increase their efficacy.

### SUMMARY OF THE INVENTION

The present invention is based on the findings that immune tolerance to an antigen can be induced by administration of the antigen incorporated into lipidic particles comprising phosphatidyl serine (PS) and phosphatidylcholine (PC), or phosphatidylinositol (PI) and PC, or in lipidic solutions. Induction of immune tolerance can be measured by an increase in TGF-β or IL-10 levels and/or a reduction in IL-6, IL-17 cytokines or co-stimulatory signals CD40, CD80 or CD86.

Accordingly, the present invention provides a method for inducing immune tolerance toward an antigen. The method comprises identifying an individual who has immune intolerance to an antigen; preparing lipidic nanoparticles comprising the antigen and a phospholipid composition selected from the group consisting of: i) PC:PI ratio of 40:60 to 60:40 (with or without 1-33% cholesterol), ii) PS:PC ratio of 10:90 to 30:70, and iii) OPLS; and
c) administering the lipidic nanoparticles to the individual. The administration results in inducing immune tolerance in the individual.

The present invention also provides stabilized PI particles which are stabilized by suspension in a buffer containing 100 to 400 nM NaCl and optionally 0.1 to 1.0 nM calcium.

### BRIEF DESCRIPTION OF THE FIGURES

Figure 1(A): Effect of phophatidylinositol on the Immunogenicity of rFVIII. (a, c), the mean of total antibody titers (horizontal bars) and individual (open circles) antibody titers were determined following s.c. and i.v. administrations, respectively. (b, d), the mean of inhibitory titers (horizontal bars) and individual (open circles) inhibitory titers were determined following s.c. and i.v. administrations.
Figure 1(B): Processing and presentation of FVIII and EPO by Dendritic cells in the presence and in the absence of lipid particles (PI, PC and PG) by measurement of MHCII, CD86 and CD40.
Figure 1(C): Dendritic cell uptake of PI and cationic lipid containing particles as studied by fluorescence microscopy (bright field (column 1), total (column 2) and intracellular fluorescence (column 3 3) of PI (row 1), DOTAP (row 2), PG (row 3) and PC (row 4).
Figure 1(D): In vitro cytokine analysis using Dendritic cells and CD4+ T-cells (a) TGF beta, (b) IL-6 and (c) IL-17.
Figure 2(A): Influence of phosphatidylinositol on pharmacokinetics of Factor VIII in Hemophilia A mice.
Figure 2 (B): Influence of phosphatidylinositol on pharmacokinetics of EPO in rats.
Figure 3: Flow-cytometry analysis of the phenotypic maturation of bone marrow-derived DCs following stimulation with PS, PC and PG liposomes associated with or without FVIII. The cell-surface markers were identified by FITC-MHC II antibody (3A), PE-CD86 antibody (3B) or PE-CD40 antibody (3C).
Figure 4: Effect of Phosphatidylserine containing formulations on the development of antibodies: (A: Development of Total antibodies; B: Development of inhibitory anti-rFVIII antibodies.
Figure 5: Influence of Phosphatidlserine on T-cell proliferation: A: CD4+ T-cell clonal expansion for liposomal formulations; B: CD4+ T-cell clonal expansion for solution-state formulations.
Figure 6A: PS mediated inhibition of T-cell proliferation as measured by 3H-thymidine incorporation. T-cell proliferation was measured for CD4+ T-cells isolated from animals immunized by sub-cutaneous (sc) administration of FVIII and re-stimulated in vitro with DCs that were exposed to FVIII in the absence or presence of liposomes (PS or PC).B: CD4+ T-cell repertoire study of solution-state formulations.
Figure 6B: OPLS mediated inhibition of T-cell proliferation as measured by 3H-thymidine incorporation. T-cell proliferation was measured for CD4+ T-cells isolated from animals immunized by sub-cutaneous (sc) administration of FVIII and re-stimulated in vitro with DCs that were exposed to FVIII in the absence or presence of liposomes (OPLS or PChg).
Figure 7: A-D PS mediated modulation of cytokine secretion as measured by ELISA. Cytokine secretion of TGF-β (7A), IL-10 (7B), IL-6 (7C) and IL-17 (7D) was measured following co-culturing of CD4+ Tcells isolated from FVIII-immunized animals with naive DCs exposed to FVIII in the absence or presence of liposomes (PS, PC and PG).
Figure 8A-D: OPLS mediated modulation of cytokine secretion as measured by ELISA. Cytokine secretion of TGF-β (8A), IL-10 (8B), IL-6 (8C) and IL-17 (8D) was measured following co-culturing of CD4+ T-cells isolated from FVIII immunized animals with naive DCs exposed to FVIII in the absence or presence of liposomes (OPLS, PChg and oPDS).

### DESCRIPTION OF THE INVENTION

The present invention provides compositions comprising lipidic particles comprising one or more antigens incorporated therein such that the immunogenicity of the antigen is reduced and or such that immune tolerance is developed toward that antigen. The present invention also provides lipidic solutions comprising one or more antigens such that the immunogenicity of the antigen is reduced and or such that immune tolerance is developed toward that antigen. It is considered that these lipid compositions provide reduced immunogenicity toward the antigen incorporated therein by altering the presentation and processing of the therapeutic protein by the immune system.

The lipid particles of the present invention comprise phospholipids such as phosphatidylserine (PS) or phosphatidylinositol (PI) in combination with Phosphatidylcholine (PC) or lipidic solutions comprising O-phospho-L-serine (OPLS). The phospholipids can be obtained from various sources both natural and synthetic. Soy PI and egg PC and PS are available commercially. Additionally, synthetic PC and PI are also available commercially.

In one embodiment, the lipidic particles comprise, consist essentially of, or consist of the phospholipids PC and PI. Cholesterol may also be present, but is not necessary for induction of immune tolerance. However, when release of the antigen is desired, it is preferable to include 1-33% cholesterol (percent of PC and PI together). Accordingly, in one embodiment, the only phospholipids or lipids are PC and PI present in the ratio of 40:60 to 60:40 and all ratios therebetween, without cholesterol. In another embodiment, PC, PI and Cholesterol are present such that the ratio of PI to PC is from 40:60 to 60:40 including all ratios therebetween and cholesterol is from 1 to 20% of the PI and PC together including all integers therebetween. In one embodiment, the cholesterol is from 5 to 15% of PC and PI together including all integers therebetween. Generally, it is considered in the art that if PI in the liposomes or lipidic particles is increased to 30% or above, the particles becomes unstable. However, in the present invention, it was unexpectedly observed that if the calcium concentration of the composition comprising the lipidic particles is between 0.1 to 1 mM and all concentrations therebetween to the tenth decimal place, preferably between 0.1 to 0.33 mM such as from 0.2 to 0.3 mM, and NaCl concentration is from 100 to 400 mM, preferably from 150 to 300 mM, the lipidic particles are stabilized. The term stabilized as used herein means that the particles are present as individual particles. If the concentration of NaCl is decreased or the concentration of Calcium is increased above the indicated values, the particles tend to fuse together and aggregate. Such aggregation is readily apparent by visual inspection because the suspension becomes turbid and the aggregated lipidic particles precipitate to the bottom. However, stabilized particles as described above can remain suspended for at least two weeks. These particles can also be lyophilized and stored in the freezer for at least 3 years. The particles comprising PI and PC as described above are referred to herein as PI particles, PI liposomes, PI lipidic particles or PI nanoparticles.

In another embodiment, the phospholipids in the lipidic particles comprise or consist essentially of, or consist of PS and PC present in the ratio of 10:90 to 30:70 and all ratios therebetween. In one embodiment, the only phospholipids in the lipidic particles are PS and PC as described above. These particles are referred to herein as PS liposomes or PS particles, PS lipidic particles or PS nanoparticles.

In another embodiment, the phospholipid in the lipidic composition comprises, consists essentially of, or consists of OPLS. In one embodiment, the only phospholipids in the lipidic particles is OPLS. The OPLS concentration can be from 1 to 100 nM including all integers therebetween. In one embodiment, it is between 5 and 25 nM including all integers therebetween. It was observed that the induction of immune tolerance was observed with OPLS but not with O-phopho-D-serine (OPDS).

The acyl chain in the phospholipids may be a diacyl chain or a single acyl chain. In one embodiment, the phospholipids PG, PS, PC and PI have two acyl chains. The length of the acyl chains attached to the glycerol backbone varies in length from 12 to 22 carbon atoms. The acyl chains may be saturated or unsaturated and may be same or different lengths. Some examples of acyl chains are: Lauric acid, Myristic acid, Palmitic acid, Stearic acid, Arachidic acid, Behenic acid, Oleic acid, Palmitoleic acid, Linoleic acid, and Arachidonic acid.

The compositions can be delivered by any standard route such as intravenous, intramuscular, intraperitonial, mucosal, subcutaneous, transdermal, intradermal, oral or the like. It is preferable to inject PS lipidic particles and OPLS compositions by subcutaneous route. However, the PI containing particles can be injected intravenously or by subcutaneous route.

The lipidic particles of the present invention can be prepared by thin lipid film hydration using the appropriate molar ratios of PC, PI and cholesterol; or appropriate ratio of PS and PI in a suitable buffer. The lipids are dissolved in chloroform and the solvent is dried. The resulting multilamellar vesicles (MLVs) are extruded through the desired size filters (sizing device) under high pressure to obtain lipidic structures of the present invention. In one embodiment, the size of 50, 60, 70, 80, 90, 95 or 100% (including all percentages between 50 and 100) of the lipidic particles is from 40 nm to 4 micron including all sizes therebetween in the nanometer and micrometer range. In another embodiment, size of the particles is from 60 to 140 nm. In one another embodiment the particles are less than 140 nm (as calculated from micrographs and dynamic light scattering measurements) so that the particles are not filtered out in the Reticulo Endothelial System (RES) so as to become available for the immune system reaction. Thus in one embodiment, at least 50% of the particles are less than 140 nm. For example, the particles are less than 120 nm such as from 40 and 100 nm. In various embodiments, 50, 60, 70, 80, 90, 95 or 100% of the particles are less than 140 nm such as from 40 and 100 nm or 60 to 100 nm.

To effect association of the protein with the lipidic structures, the protein in a suitable buffer is added to the lipidic structures. The free protein is then separated from the lipidic structures by centrifugation methods such as density gradient centrifugations. In various embodiments, the association efficiency of the protein with the lipidic particles is at least 30, 40, 50, 55, 60, 65, 70, 75, 80, 85, 90 and 95%. If desired, the lipidic particles with the associated antigen can be lyophilized for future use.

In one embodiment, the lipidic structures of the present invention prior to association with the protein can be lyophilized and stored. When needed, the lipidic structures can be reconstituted and then used for combination with protein to effect association of the protein with the lipidic structures prior to use. For the PI:PC/Cholesterol particles, the reconstitution is preferably done in a solution containing 0.1 to 1.0 mM calcium and 100 to 400 mM NaCl. In one embodiment, while NaCl is from 100 to 400 mM, there is no calcium present.

The antigen may be a peptide (generally 50 amino acids or less) a polypeptide (generally 100 amino acids or less) or proteins (larger than 100 amino acids). The agent may be a therapeutic antigen or may be an antigen against which an individual is already primed, but against which immune tolerance is desired (such as in allergic reactions or transplant situations). The proteins or peptides may be neutral or charged (negatively or positively). Such proteins include proteins involved in the blood coagulation cascade including Factor VIII (FVIII), Factor VII (FVII), Factor IX (FIX), Factor V (FV), and von Willebrand Factor (vWF), von Heldebrant Factor, tissue plasminogen activator, insulin, growth hormone, erythropoietin alpha, VEG-F, Thrombopoietin, lysozyme and the like. In one embodiment, the antigen can be one that ordinarily provokes a relatively mild allergic reaction, such as would typically be caused by pollen, animal dander, house dust and the like, or an antigen that ordinarily would provoke in the individual a severe allergic reaction, such as components in venom from insect stings, nut allergens, certain antibiotics, and other compositions that can cause severe allergic responses in the particular individual in question or may be a transplant relevant antigen.

The association of the protein with the lipidic structures can be such that the molar ratio between the protein to lipid is between 1:200 (protein:lipid) to 1:30,000 (protein:lipid) and all ratios therebetween. In one embodiment it is about 1:10,000 (protein:lipid). In other embodiments, the ratio is about 1:2,000 or 1:4,000 (protein:lipid).

Immune responses against antigens involve several steps, including processing and presentation of the protein by antigen presenting cells (APCs) in the context of the Major Histocompatibilty Complex (MHC), interaction of APCs and T-cells mediated by MHC - T-cell receptor (TCR) interaction (in the presence of co-stimulatory signals and cytokine support), followed by T-cell maturation, T-cell - B-cell interaction and B-cell maturation. The method of the present invention involves induction of immune tolerance which is related, at least in part, to regulator T cells (T_{regs}). These cells are also referred to as suppressor T cells because they function to suppress activation of the immune system. T_{regs} are known to secrete the immunosuppressive cytokine TGF-β.

We demonstrate that the present invention facilitates an increase in TGF-β secretion by T_{regs} and that this is associated with a reduction in IL-6, IL-17 and other cytokine secretion. Additionally, co-stimulatory signals (CD40, CD8 and CD86) are also decreased. Without intending to be constrained by theory, it is believed that one or more of these effects are at least in part responsible for induction of immune tolerance and/or a reduction in antibody titer. It is further believed that the induction of immune tolerance and/or reduction in antibody titer is related to T_{reg} expansion which results in a durable, specific immune tolerance to the antigen that is administered to an individual as a component of a composition of the invention. It is therefore considered that the method of the invention is suitable for inducing immune tolerance and/or reducing the titer of antibodies produced by an individual when exposed to an antigen, and is expected to be particularly suited for reducing antibodies specific for an antigen to which the individual has previously developed, or is at risk for developing, an allergic response. Therefore, in various embodiments, the invention provides for reducing antibody titers in an individual, wherein the antibodies are specific for an antigen that is prone to causing an allergic reaction in the individual. The allergic reaction can be mild, such as a hayfever or a skin rash, or severe, such as constriction of airways and/or anaphylaxis.

We have also observed that *in vitro,* PI interfered with the processing of an antigen (FVIII) by cultured dendritic cells as observed by a reduction in the up-regulation of phenotypic co-stimulatory signals CD40 and CD86. Furthermore, PI increased secretion of regulatory cytokines Transforming Growth Factor beta1 (TGF-β1) and Interleukin 10 (IL-10) but reduced the secretion of pro-inflammatory cytokines IL-6 and IL-17.

Additionally, we have also observed that when mice were subjected to four weekly injections of antigen (FVIII) alone or incorporated into PI nanoparticles, and spleens removed after another 2 weeks, the percent of CD4 and CD25 T_{reg} cells as a percent of total lymphocytes in PI-nanoparticles was about 20%.

Thus, the data presented herein provides support that PI reduces the immunogenicity of antigens by modulating DC maturation and by secretion of regulatory cytokines. It is believed that the lipidic nanoparticles described herein provide immune tolerance at least in part by reducing immunogenic T cells to tolerogenic T cells.

In one embodiment, the invention provides a method for reducing antibody titer against an antigen in an individual comprising the steps of: a) identifying an individual who has a high titer of antibodies to the antigen; b) preparing stabilized lipidic nanoparticles comprising the antigen and the one of the following: i) PC:PI ratio of 40:60 to 60:40; ii) PS:PC ratio of 30:70 to 10:90; or preparing lipid solution comprising the antigen and OPLS; and c) administering the stabilized lipidic nanoparticles or the lipidic composition to the individual. The administration results in reducing the titer of said antibodies. The PC:PI nanoparticles may contain 1-33% cholesterol. In one embodiment, the particles have 5-15% cholesterol.

In another embodiment, the invention provides a method for inducing immune tolerance toward an antigen comprising the steps of: a) identifying an individual who has immune intolerance to an antigen; b) preparing stabilized lipidic nanoparticles comprising the antigen and a phospholipid composition which can be: i) PC:PI ratio of 40:60 to 60:40 which has no cholesterol or has cholesterol from 1 to 33 %, 1 to 20%, or 5-15% and all integers therebetween; ii) PS:PC ratio of 10:90 to 30:70 and all ratios therebetween; or preparing a lipidic composition comprising the antigen and OPLS; c) administering the stabilized lipidic nanoparticles to the individual. Such administration results in inducing immune tolerance in the individual toward said antigen. Immune tolerance is generally considered as the active induction of an antigen specific immunological non responsiveness. Reduction in immune tolerance can be evidenced by one or more of the following: i) reduction in antibody titer relative to the titer present prior to administration, ii) increase in TGF-b and/or IL-10 levels; iii) decrease in one or more cytokines such as IL-6, IL-17, and/or co-stimulatory signals such as CD40, CD80, CD86,

In another embodiment, the invention provides a composition comprising: stabilized lipidic nanoparticles comprising an antigen incorporated into a lipidic nanoparticle, wherein the phospholipids of the lipidic structure are PC:PI ratio of 40:60 to 60:40, with or without cholesterol. If cholesterol is present, it is present between 1 to 20% and all integers therebetween. In one embodiment, the cholesterol is 5 to 15%. The particles are stabilized by having a calcium concentration of 0.1 to 1.0 mM and NaCl concentration of 100 to 400 mM. In one embodiment, the calcium concentration is 0.15 to 0.35 mM and NaCl is from 100 to 300 mM.

### ADDITIONAL DISCLOSURES

Additionally disclosed is:
Paragraph 1. A method for inducing immune tolerance toward an antigen comprising the steps of:
   a) preparing lipidic particles comprising the antigen and a phospholipid composition selected from the group consisting of:
      i) PC:PI in a ratio of 40:60 to 60:40, and
      ii) PS:PC in a ratio of 10:90 to 30:70; and
   b) administering the lipidic particles to an individual who has immune intolerance to the antigen,
      wherein the administration results in inducing immune tolerance in the individual to the antigen and wherein the antigen is a protein, polypeptide or peptide.
Paragraph 2. The method of paragraph 1, wherein induction of immune tolerance is evidenced by one or more of the following:
   i) reduction in antibody titer relative to the titer present prior to administration,
   ii) increase in TGF-β and/or IL-10 levels
   iii) reduction in one or more of the following: CD40, CD80, CD86, IL-6, IL-17.
Paragraph 3. The method of paragraph 1, wherein the composition of i) further comprises cholesterol such that cholesterol is 1-20% of PC and PI together.
Paragraph 4. The method of paragraph 3, wherein the cholesterol is 5-15% of PC and PI together.
Paragraph 5. The method of paragraph 1, wherein the composition comprising PC and PI further comprises from 100 to 400 mM NaCl.
Paragraph 6. The method of paragraph 5, wherein the composition further comprises from 0.1 to 1.0 mM calcium.
Paragraph 7. The method of paragraph 1, wherein the ratio of PC:PI is 45:55 to 55:45.
Paragraph 8. The method of paragraph 1, wherein the at least 50, 60, 70, 80 or 95% of the particles are from 60 to 140 nm.
Paragraph 9. The method of paragraph 1, wherein the antigen associated with the lipidic particles is present in the ratio of 1:2,000 to 1:4,000.
Paragraph 10. A method for inducing immune tolerance toward an antigen comprising the steps of:
   a) preparing lipidic composition comprising the antigen and OPLS; and
   b) administering the lipidic composition from a) to an the individual who has immune intolerance to an antigen,
   wherein the administration results in inducing immune tolerance in the individual to the antigen, wherein the antigen is a protein, polypeptide or a peptide.
Paragraph 11. The method of paragraph 10, wherein induction of immune tolerance is evidenced by one or more of the following:
   i) reduction in antibody titer relative to the titer present prior to administration,
   ii) increase in TGF-β and/or IL-10 levels
   iii) reduction in one or more of the following: CD40, CD80, CD86, IL-6, IL-17.
Paragraph 12. A composition comprising stabilized lipidic nanoparticles comprising an antigen incoporated therein, wherein the phospholipids of the lipidic nanoparticles are selected from the group consisting of PC:PI ratio of 40:60 to 60:40 and wherein the lipid nanoparticles are stabilized by a buffer containing sodium chloride from 100 to 400 mM.
Paragraph 13. The composition of paragraph 12, further comprising 0.1 to 1.0 mM calcium.
Paragraph 14. The composition of paragraph 13, wherein the NaCl is from 150 to 300 mM.
Paragraph 15. The composition of paragraph 14, wherein the calcium is from 0.15 to 0.35 mM.

### EXAMPLE 1

### Methods

Materials: Albumin free full-length (Baxter Health Care Glendale, CA) and B-domain deleted rFVIII (Wyeth, St Louis MO and American Diagnostica, Greenwich, CT) were used for studies. Advate, Refacto and Novoseven were provided as a gift from Western New York Hemophilia foundation. Albumin free Recombinant EPO was purchased from Prospec Inc, Israel. Dimyristoyl phosphatidylcholine (DMPC) and soybean phosphatidylinositol (Soy PI) were purchased from Avanti Polar Lipids (Alabaster, AL). Cholesterol, IgG-free bovine serum albumin (BSA), and diethanolamine were purchased from Sigma (St. Louis, MO). Goat antimouse-Ig and antirat-Ig, alkaline phosphatase conjugates were obtained from Southern Biotechnology Associates, Inc. (Birmingham, AL). p-Nitrophenyl phosphate disodium salt was purchased from Pierce (Rockford, IL). Monoclonal antibodies ESH4, ESH5, and ESH8 were purchased from American Diagnostica Inc. (Greenwich, CT). Monoclonal antibody N77210M was purchased from Biodesign International (Saco, ME). Normal coagulation control plasma and FVIII-deficient plasma were purchased from Trinity Biotech (County Wicklow, Ireland). The Coamatic FVIII kit from DiaPharma Group (West Chester, OH) was used to determine the rFVIII activity in plasma samples.

Preparation of Protein- Lipid (PI, PC and PG) particles: Lipidic particles were prepared by hydration of thin lipid film in appropriate molar ratios of the phospholipids. For example, DMPC, ±soy PI/ Dimyristoyl PhosphatidylGlycerol DMPG, and cholesterol (50:50:5) with Tris buffer (TB) (25mM Tris, and 300mM NaCl, pH=7.0). PI particles were made with PI:PC/cholesterol 50:50:5; PC particles were made with 100% PC; and PG particles were made with PG:PC 30:70). The required amount of lipid was dissolved in chloroform in a kimax tube and the solvent was dried using Buchi-R200 rotaevaporator (Fisher Scientific, NJ). Multilamellar vesicles (MLV) were formed by mixing the lipid dispersions at 37°C for 20 min. The resulting MLV were extruded through double polycarbonate membranes of 80nm pore size (GE Osmonics Labstore, Minnetonka, MN) in a high-pressure extruder (Mico, Inc., Middleton, WI) at a pressure of ∼250 psi and then sterile-filtered through a 0.22 m MillexTM-GP filter unit (Millipore Corporation, Bedford, MA). Phosphate assay was performed to estimate concentration of phospholipid and its recovery. Particle size was monitored using a Nicomp Model CW 380 particle size analyzer (Particle Sizing Systems, Santa Barbara, CA) and lipid organization and dynamics of the particle was investigated using biophysical studies. The protein was added to lipidic particles at 37°C and during this process Ca2+ ion concentration was adjusted in TB to ensure optimal lipid-Ca2+ interaction and lipid phase change. It is appropriate to mention here that presence of surfactants, carrier protein and other organic solvents can interfere with loading of the protein in the particle. The protein to lipid ratio was maintained at 1:10,000 and total protein and lipid concentrations were same for all experiments, unless stated otherwise.

Separation of Free Protein from Protein-lipid Complexes: Discontinuous dextran density gradient centrifugation technique was used to separate the free protein from particle associated protein to estimate the amount of protein associated with the particle. Spectroscopic assay and one-stage activated partial thromboplastin time (aPTT) assay was used to estimate the association efficiency of FVIII and spectroscopy based assay to estimate the concentration of EPO associated with the particle. For in vivo studies, the free protein was not separated from particle bound protein to avoid contamination in immunogenicity studies. Further, for need basis therapy as in the case of Hemophilia A, free and loosely bound protein is required for clotting during bleeding episodes and a prolonged exposure from tightly bound protein to reduce frequency of administration.

Animals: Breeding pairs of Hemophilia A mice (C57BL/6J) with a target deletion in exon of the FVIII gene were used. A colony of Hemophilia A mice was established and animals aged from 8-12 weeks were used for the in vivo studies. Since the sex of the mice has no impact on the immune response, both male and female mice were used for the immune response studies. Wild type C57BL/6J mice was used for immunogenicity studies with EPO. Male wistar rats weighing 300-350g was used for pharmacokinetics studies involving EPO.

In Vivo Activity of rFVIII-PI and EPO-PI: Tail clip method was used in Hemophilia A mice to investigate rFVIII-PI activity in vivo. Animals (n=3) were administered with rFVIII alone or rFVIII-PI by intravenous route. Briefly, 2 g (∼10IU) of rFVIII was given per 25 g of animal body weight. Forty eight hours post injection, tip (1 cm) of the tail was cut off using a scalpel and the survival of the animals was monitored. The efficacy of EPO and EPO-PI was tested in male wistar rats. All rats received single dose of 450 IU/kg via tail vein for i.v. bolus administration (referred as day 0). Blood samples (75 µl) were collected from tail vein at day 0 (predose), 1, 2, 3, 4, 5, 6, 7, 8, 9, 10, 11, 12, 13, 23 days after the injections. Blood samples were collected with EDTA and analyzed within 2 hr of collection. RET counts were obtained using flow cytometry (FACS Calibur; BC Biosciences, Franklin Lakes, NJ) with thiazole orange. The peak RET response (RETmax) normalized to their baseline (RETo) as RETmax/RETo, was used as a quantitative measurement for assessment of therapeutic efficacy. RETmax/RETo represents the ratio between peak of the absolute RET count in response to the treatment and the endogenous absolute RET count of the rats. Higher value of RETmax/RETo would reflect a greater extent of production of RET resulting from higher activity of bone marrow.

Immunogenicity Studies: The relative immunogenicity of free rFVIII and rFVIII-PI were determined in Hemophilia A mice. 8 male and 10 female mice received 4 weekly intravenous injections (via penile vein) and subcutaneous injections, respectively. Two weeks after the last injection, blood samples were collected in acid citrate dextrose (ACD) buffer (85mM sodium citrate, 110mM D-glucose and 71mM citric acid) at a 10:1 (v/v) ratio by cardiac puncture. Plasma was separated by centrifugation at 5,000 rpm at 4°C for 5min. Samples were stored at - 80°C immediately after centrifugation. Total anti-rFVIII antibody titers were determined by standard antibody capture ELISA. Inhibitory antibody titers were measured by the Nijmegen modification of the Bethesda assay.

Isolation and Characterization of dendritic cells: Dendritic cells were isolated from the bone marrow of hemophilic mice as described previously by Lutz et al. This prolonged culture procedure reduced granulocyte contamination and yields a purity of over 70% (that comprised of mature and immature dendritic cells). In order to verify the differentiation of bone marrow cells into dendritic cells and its purity, the expression of DC markers MHC II, CD11c, CD40, CD80 and CD86 were monitored using flow cytometry. DCs thus obtained are mixtures of immature and mature and the percentage of immature (iDCs) was determined by maturation using Lipopolysaccharide (LPS), a well known strong antigen. Measuring the shift in peak in flow cytometry analysis before and after incubating with LPS, confirmed the presence of over 50% of iDCs in culturing conditions. Hemophilia A mice and wild type C57BL/6J mice were used for FVIII and EPO studies respectively. Brief description of the procedure; the hind limbs which include the femurs and tibiae were obtained and any attached muscle or skin was removed carefully so as not to break the bones. The bones were placed in 70% alcohol for disinfection. The bones were later placed in a sterile petri-plate in a cell culture hood. The ends of the bones were cut and ice-cold, sterile and iso-osmotic PBS at pH 7.0 was flushed with the help of a 10ml sterile syringe with a 25 gauge needle. The flushed bone marrow was collected in the Petri plate. The cells were pipetted gently and passed through a cell strainer to loosen any cell clumps as well as to remove debris. The cell suspension was centrifuged at 300g for 10 minutes at 40°C. The supernatant was discarded and the cells were resuspended in residual medium. Viable cells were counted under a microscope using the hemocytometer and trypan-blue method. Accordingly, 2 x 10⁶ cells were plated in a sterile petri-plate along with 1ml of sterile FBS, 4uL of 200units/ml of rmGMCSF and RPMI-1640 media containing Penicillin-Streptomycin, 2-mercaptoethanol and L-Glutamine, making the total volume up to 10ml. This was considered as day '0'. The cells were incubated at 37C and 5% CO2. On day 3, another 10ml of media containing rmGMCSF and FBS was added to the petri-plate. On day 6 and 8, 10ml of the supernatant from the petri-plate was aspirated and centrifuged in a 15ml centrifuge tube at 300g for 10 minutes at 40C. Supernatant was discarded and the cell pellet was resuspended in 10ml of fresh medium containing rmGMCSF and FBS. On day 10, cells were harvested by gentle pipetting. The cells thus obtained were further used for characterization and other experimental studies. This prolonged culture procedure with reduced GMCSF concentration by Lutz et al reduces granulocyte contamination and yields a purity of over 90% (that comprised of mature and immature Dendritic cells). In order to verify the differentiation of bone marrow cells into dendritic cells and its purity, the expression of DC surface markers MHC II, CD11c, CD40, CD80, and CD86 were monitored using flow cytometry. DCs thus obtained are mixtures of immature and mature and the percent of immature (iDCs) was determined by maturation using Lipopolysaccharide (LPS), a well known strong antigen. Measuring the shift in peak in flow cytometry analysis before and after incubating with LPS, confirmed the presence of over 50% percent of iDCs in culturing conditions.

Processing and presentation of antigen and Dendritic Cells maturation studies: Day 9 naive immature BMDCs from hemophilic mice were sub-divided into different groups in 6-well plates. The cells were co-cultured with free FVIII, FVIII-lipid particles/liposomes and blank liposomes in growth media. The co-culture was incubated for 24 hr at 37C and 5 % CO2. After 24 hr, the different groups of cells were harvested separately and washed with 1X PBS. Viable cells were counted using hemocytometer and trypan blue. Accordingly, 1x10⁶ viable DCs were added to flow-cytometry tubes. The cells were washed again and incubated with Fc-Block antibody on ice. Later, the cells were labeled with fluorescently tagged anti-MHC-II, anti-CD86 or anti-CD40 antibodies on ice. Appropriate isotype controls were also used in the study. Finally, 0.5ml of 2% ultrapure paraformaldehyde was added to each tube. The levels of the different cell surface markers were determined using BD^{©} FACS Calibur flow-cytometer. Similar set of study was carried out for rhEPO but the BMDCs were obtained from wild type C57BL/6J mice.

Dendritic cell uptake studies: HPTS-Containing Liposome Preparation: Lipid films (10 umol) were rehydrated and vortexed in a 35 mM HPTS solution. The resulting vesicles were extruded through double polycarbonate filters with pore diameter of 0.1 um. The HPTS-containing liposomes were separated from the free HPTS using a Sephadex G-75 column (1 X 30 cm). The column was equilibrated with the Tris buffer (140 mM NaCl, 25 mM Tris, pH 7.4). Each liposome preparation was loaded to the column and 0.5 ml of eluent was collected for each fraction. 200 ul of each fraction was added to the 96 well plate and the fluorescence intensity was determined using a microplate spectrofluorometer (Spectra Max Gemini, Molecular Devices, Sunnyvale, CA). The sample was excited at 413 nm and the fluorescence emission was detected at 515 nm. Fraction numbers and fluorescence intensity were plotted in linear or log scale and was used to select HPTS-containing liposomes for the dendritic cell uptake study. Phosphate assay was performed to estimate concentration of phospholipid and its recovery. Dendritic Cell Uptake Study: Dendritic cells were harvested on day 8 of the dendritic cell culture. Briefly, culture supernatant was collected and centrifuged at 300 x g for 10 min at 4C. The cell pellet was resuspened in media and counted under the microscope using a hemocytometer in the presence of Trypan Blue solution (1:1 cell aliquot to Trypan Blue volume). 1x10⁶ cells in 4 ml of DC media were seeded per 35 x 10mm tissue culture dish (Becton Dickinson and Company, Franklin Lakes, NJ) that contained a pre-sterilized 22 x 22mm micro cover glass (No. 1.5). Cells would adhere to the cover glass after 24 hrs of culture and were used for the uptake study. Cells grown on coverslips were washed three times with Tris buffer (140 mM NaCl, 25 mM Tris, 0.36 mM CaCl2, 0.42 mM MgCl2, pH 7.4) and then were treated with HPTS-containing liposomes. Briefly, 500 uM of liposome preparation (0.1 umol/200 ul) was added to each coverslip of 10⁶ cells in the dish. Cells were incubated for 30 min at 37C in the humidified incubator, and were washed three times with Tris buffer. The cell-grown coverslip was carefully mounted (cells facing down) onto a 3" x 1" x 1.0mm microscope slide. Fluorescence Microscopy:

Liposome/Dendritic Cell Interaction: HPTS-Containing Liposome Preparation: Lipid films (10µmol) were rehydrated and vortexed in a 35 mM HPTS solution. The resulting vesicles were extruded through double polycarbonate filters with pore diameter of 0.1 *u*m. The HPTS containing liposomes were separated from the free HPTS using a Sephadex G-75 column (1 X30 cm). The column was equilibrated with the Tris buffer (140 mM NaCl, 25 mM Tris, pH 7.4). Each liposome preparation was loaded to the column and 0.5 ml of eluent was collected for each fraction. 200 ul of each fraction was added to the 96 well plate and the fluorescence intensity was determined using a microplate spectrofluorometer (Spectra Max Gemini, Molecular Devices, Sunnyvale, CA). The sample was excited at 413 nm and the fluorescence emission was detected at 515 nm. Fraction numbers and fluorescence intensity were plotted in linear or log scale and was used to select HPTS-containing liposomes for the dendritic cell uptake study. Phosphate assay was performed to estimate concentration of phospholipid and its recovery. Dendritic Cell Uptake Study: Dendritic cells were harvested on day 8 of the dendritic cell culture. Briefly, culture supernatant was collected and centrifuged at 300 x g for 10 min at 4C. The cell pellet was resuspened in media and counted under the microscope using a hemocytometer in the presence of Trypan Blue solution (1:1 cell aliquot to Trypan Blue volume). 1x10⁶ cells in 4 ml of DC media were seeded per 35 x 10mm tissue culture dish (Becton Dickinson and Company, Franklin Lakes, NJ) that contained a pre-sterilized 22 x 22mm micro cover glass (No. 1.5). Cells would adhere to the cover glass after 24 hrs of culture and were used for the uptake study. Cells grown on coverslips were washed three times with Tris buffer (140 mM NaCl, 25 mM Tris, 0.36 mM CaCl2, 0.42 mM MgCl2, pH 7.4) and then were treated with HPTS-containing liposomes. Briefly, 500 *u*M of liposome preparation (0.1 *u*mol/200 *u*l) was added to each coverslip of 106 cells in the dish. Cells were incubated for 30 min at 37C in the humidified incubator, and were washed three times with Tris buffer. The cell-grown coverslip was carefully mounted (cells facing down) onto a 3" x 1" x 1.0mm microscope slide. Liposome Uptake Experiments: PI-containing lipidic particles were labeled with 3 mole% of tetramethylrhodamine-labeled phosphatidylethanolamine (Rho-PE) and were incubated with DCs for 25 min at 37oC. In order to avoid high "background" staining, the Fc receptors (FcR) of the FcR-bearing cells were blocked with anti-mouse CD16/CD32 monoclonal antibody (0.0833 mg/ml) for 10 min at 4C. This is followed by the subsequent staining of DCs with fluorescein isothiocyanate (FITC) conjugated anti-mouse CD11c antibody (0.167 mg/ml) for 10 min at 4C. Fluorescence Microscopy: The uptake of HPTS labeled lipidic particles was monitored using Zeiss axiovert 200M inverted fluorescence microscope fitted with AxiocamMR3 camera. The pH dependent fluorescence was measured with filter set 10 (excitation band pass 450-490 nm, emission band pass 515 - 565 nm) and total fluorescence was monitored using filter set 02 (excitation 365 nm and emission long pass 420 nm) provided by the manufacturer. The rhodamine fluorescence was measured by Filter set 20 (Excitation: BP 546/12, beamsplitter: FT 560, emission: BP 575-640). The images were acquired at 20X and 63X setting and analyzed using the software Axiovision 4.6.3 provided by the manufacturer.

T-cell proliferation Studies: Two s.c. injections of rFVIII or rFVIII-PI (2 ug) were administered to female Hemophilia A mice at weekly basis. Three days after the second injection, animals were sacrificed and the spleens were harvested as a source of T-cells. To enrich CD4+ T-cells, a CD8+ T-cell depletion kit was used. CD4+ T-cells were cultured in 96 well plates with 100 ng/well rFVIII and then incubated with 3H-thymidine after 72 hr of culture. T-cells were harvested at the end of 16 hr and 3H-thymidine was measured using scintillation counter.

Cytokine Analysis: In order to study the effect of different cytokines' role on the immunogenicity of rFVIII (EPO) and rFVIII-lipid (EPO-lipid) complexes, cytokine analysis was performed.Briefly, female hemophilia A mice between 8-12 weeks old were immunized with free rFVIII via the s.c. route for two consecutive weeks. Simultaneously, another naive hemophilia A mouse was sacrificed and bone marrow was isolated from the femurs and tibiae and differentiated into immature Dendritic cells (iDCs). These iDCs were challenged with different lipidic FVIII formulations and washed. On the third day after immunization, the animals were sacrificed and their spleens were obtained. Splenic CD4+ T-lymphocytes were isolated using a commercially available isolation kit from Invitrogen Inc CA. CD4+ T-cells and iDCs that were exposed to formulations, were co-cultured in 96-well culture plates at 37°C and 5%CO2. After 72 hrs after of co-culture, the plates were centrifuged and the supernatant was collected for cytokine analysis using commercially available ELISA kit from (RnD systems, MN). As mentioned above, for recombinant hEPO studies, wild type C57BL/6J mice was used.

Pharmacokinetics Studies: rFVIII and rFVIII-PI (10 IU/25g) was administered to male Hemophilia A mice as a single intravenous bolus injection via penile vein. Blood samples were collected in syringes containing ACD buffer (10:1 v/v) at 0.08, 0.5, 1, 2, 4, 8, 16, 24, 36, and 48 hrs after the injections by cardiac puncture (n=3-6 mice/time point). Plasma was collected immediately by centrifugation (5,000 rpm, 5 min, 4C) and stored at -70C until analysis. Chromogenic assay was used to measure the activity of rFVIII in plasma samples.

For EPO and EPO-PI, Male Wistar rats weighing from 275 to 300 g (Charles River Laboratories, Inc. Raleigh, NC) were injected with 450 IU/kg as a single intravenous bolus injection via tail vein (n=3). Blood samples (100-150 µl) were collected from the tail vein at 0.08, 0.5, 1, 2, 4, 8, 12, 24, 32, and 48 h after the injections (n=3 rats/time point). Serum concentrations of rHuEPO were determined using the Quantikine IVD Epo ELISA (R&D Systems Inc., Minneapolis, MN). Since this ELISA kit is specific for rHuEPO, it did not detect the endogenous EPO in rats.

The average values of rFVIII activities and EPO concentration at each time point were used to compute basic pharmacokinetic parameters (half-life, MRT and area under the plasma activity curve) using a noncompartmental analysis (NCA) with the program WinNonlin (Pharsight Corporation, Mountainview, CA). The areas under the plasma activity (AUC) versus time curves from 0 to the last measurable activity time point were measured by log-linear trapezoidal method. The elimination rate constant (lambda z) was estimated by log-linear regression of the terminal phase concentration. The elimination half-life (t_{1/2}) was calculated as ln 2/lambda z. MRT was calculated from AUMC/AUC where AUMC is the area under the curve, plot of the product of concentration and time versus time.

Statistical Analysis: Statistical difference (p<0.05) was detected by the Student t-test, and one-way ANOVA followed by Dunnette's post-hoc multiple comparison test. One-way ANOVA with post-hoc analysis was performed using SPSS statistical software (SPSS Inc.) for cytokine analysis. For PK studies, repeated-measures ANOVA was used to compare the profiles generated by the two treatments. Bailer-Satterthwaite method was used to compare differences in systemic exposure between the two treatments.

In the present invention, FVIII was associated with PI containing lipidic-particle as described in the *Experimental Procedures* section. Unincorporated protein was separated from protein associated with lipidic particles using Dextran gradient centrifugation, and the concentration of the protein was estimated based on both activity and spectroscopic assays. This procedure yielded an association efficiency of 72 ± 9% of the added protein, and is much higher than observed with lipidic complexes containing alternative acidic (phosphatidylserine PS, Phosphatidyl glycerol PG and Phosphatidic acid PA and neutral (Phosphatidyl Choline PC) phospholipids. This finding is remarkable in that FVIII binds PS-containing membranes with high affinity, and therefore the observed higher efficiency of FVIII association with PI nanoparticles may not result from electrostatic interaction and surface adsorption alone.

It is considered that the higher association of FVIII with PI nanoparticles is because of the lipid organization and packing defects lead to distorted bilayer organization, which could increase the incorporation of the protein in the particle. In this topology, a substantial surface area of the FVIII molecule, and/or a greater number of FVIII molecules would be shielded by the PI particle, compared to the binding of FVIII to PS containing liposomes. Circular Dichroism and functional studies were carried out in order to evaluate the conformation and activity of FVIII associated with the lipidic nanoparticles. Association of FVIII with PI nanoparticles did not alter the far UV CD spectrum of the protein, and thermal stress studies indicated that nanoparticle-associated protein displayed an improved stability profile. The activity of the protein, as measured by apTT (activated prothrombin time) and by an in vitro ELISA-based chromogenic assay showed clearly that the protein retained activity upon association with the PI nanoparticle. Biophysical studies of tryptophan (Trp) fluorescence showed that interaction of FVIII with the PI nanoparticle rendered a significant fraction of Trp residues inaccessible to quenching by acrylamide.

The particle loading procedure and retention of activity was not limited to FVIII. EPO is a naturally occurring glycoprotein hormone. Recombinant human erythropoietin (rHuEPO), is used as a treatment for anemia in patients with chronic renal failure and receiving chemotherapy. Prolonged use of rHuEPO has been shown to break tolerance, whereby the antibodies bind to both endogenous and exogenous rHuEPO, leading to pure red cell aplasia. A less immunogenic therapeutic preparation of EPO will improve safety and efficacy of this therapy. In this direction, EPO was associated with PI lipidic particles. The in vivo and in vitro activity was preserved following complexation of EPO with PI nanoparticles and a substantial portion of intrinsic Trp residues were inaccessible to acrylaminde (Supplemental data). Thus, the simple particle preparation procedure leads to association and preservation of biological activity of FVIII and EPO, proteins with different physico-chemical properties.

Hemophilia A mice represent a suitable animal model in which to investigate immunogenicity. FVIII is not expressed in these mice, and the antibody response patterns against FVIII are similar to those observed in hemophilic patients. Hemophilic mice received weekly treatments with 10 IU of free FVIII or the FVIII-PI formulation by i.v. and s.c. injection (n=8 for i.v., n=10 for s.c.) for 4 weeks. Two weeks following the last injection, blood samples were collected. Anti-FVIII antibody levels were determined by ELISA and the titer of inhibitory antibody titers was determined using a modified Bethesda assay.

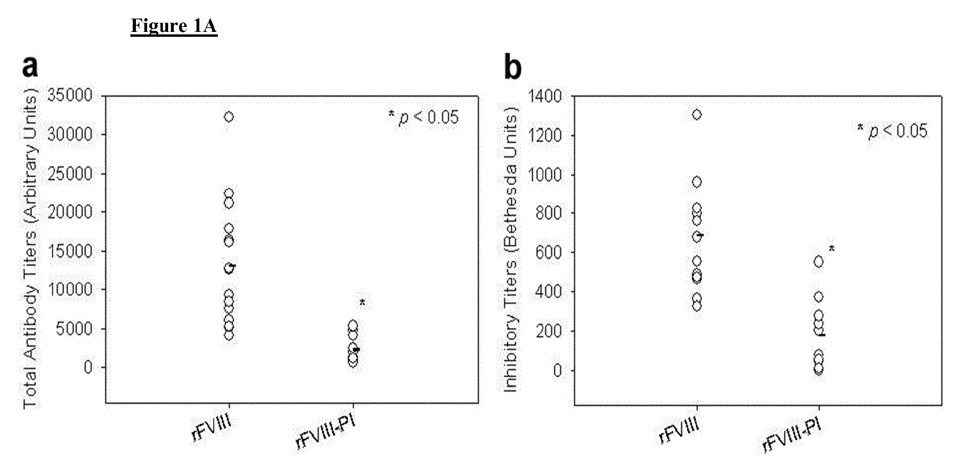

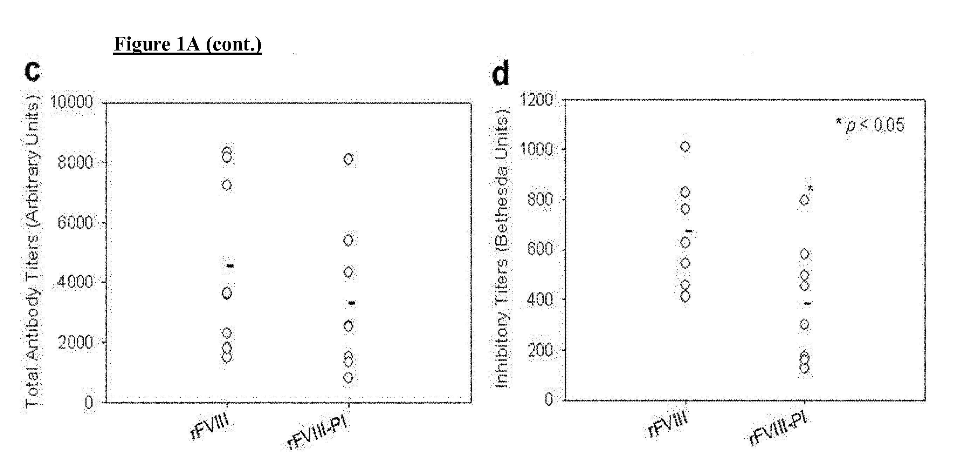

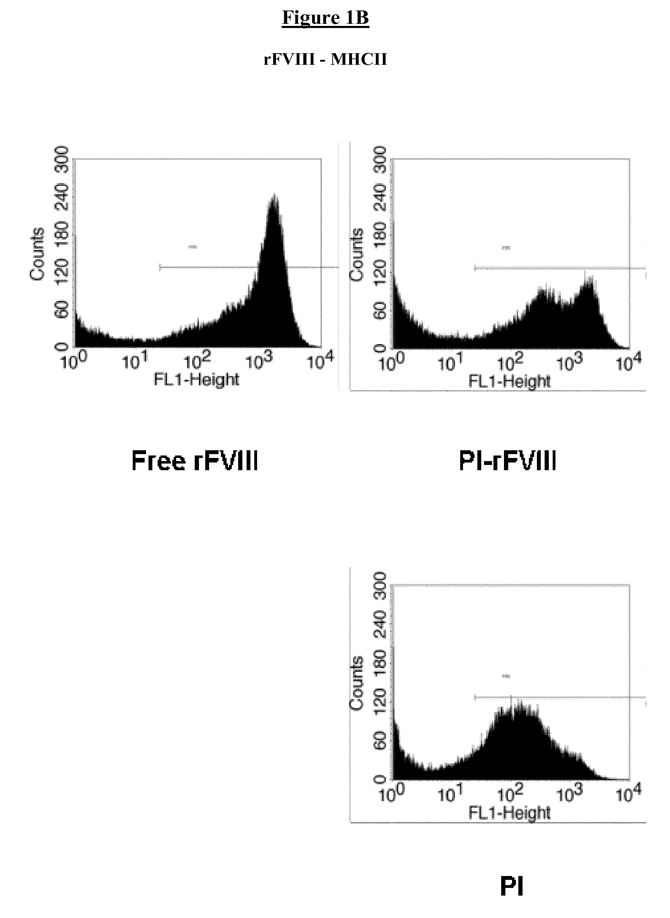

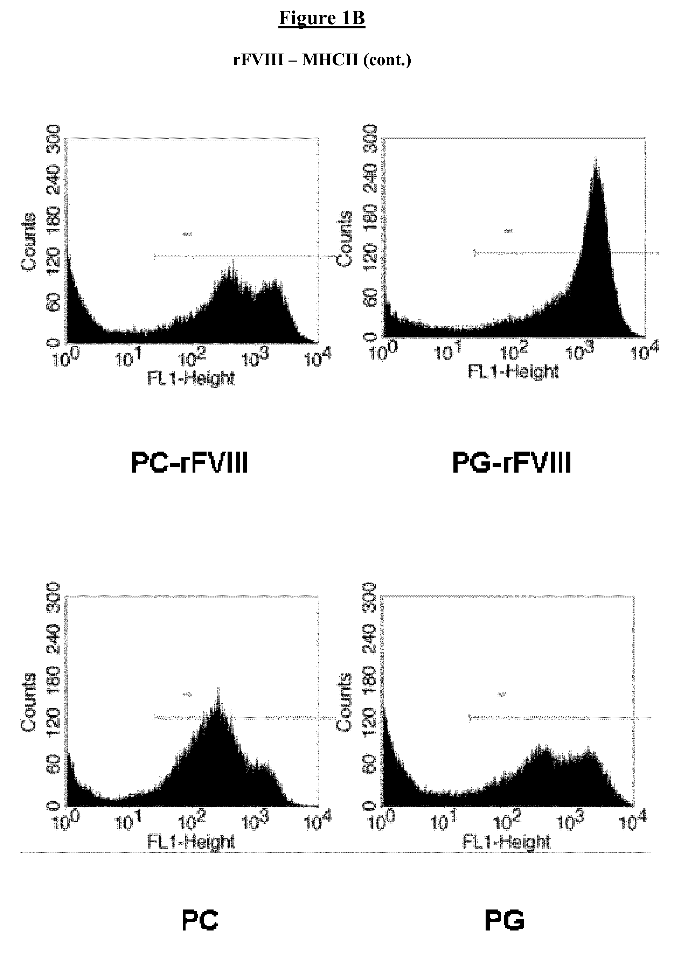

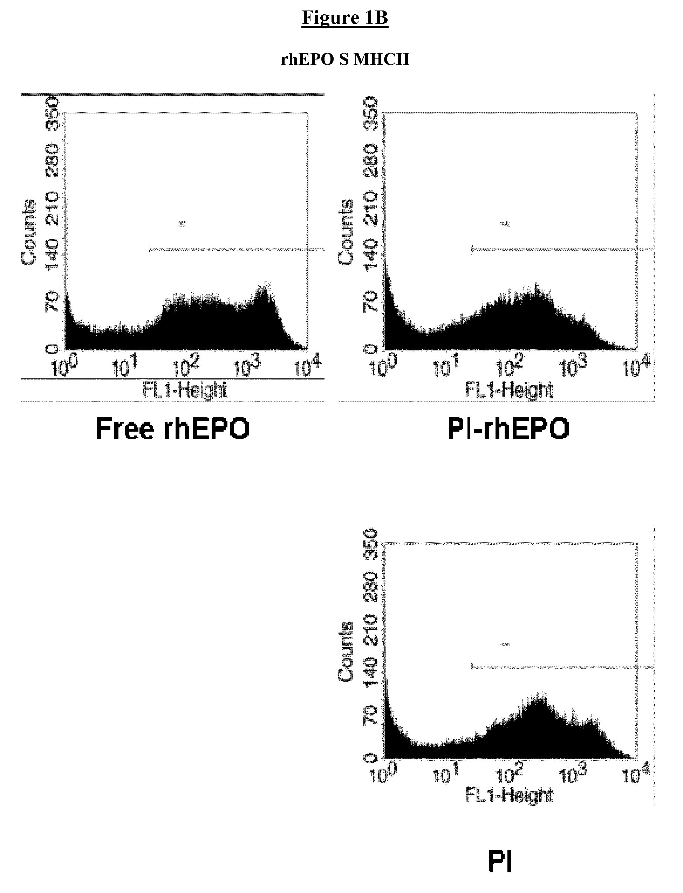

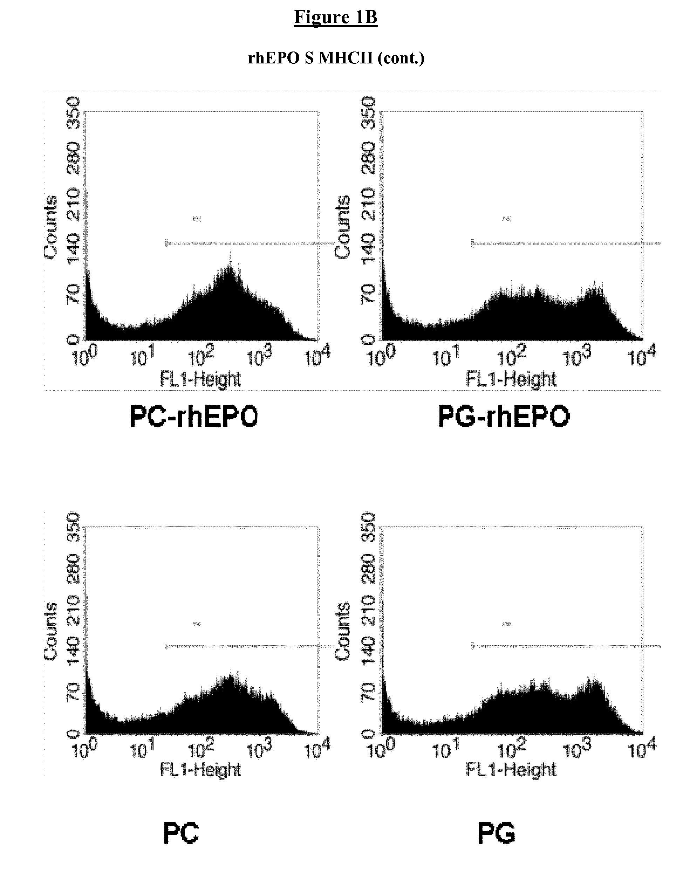

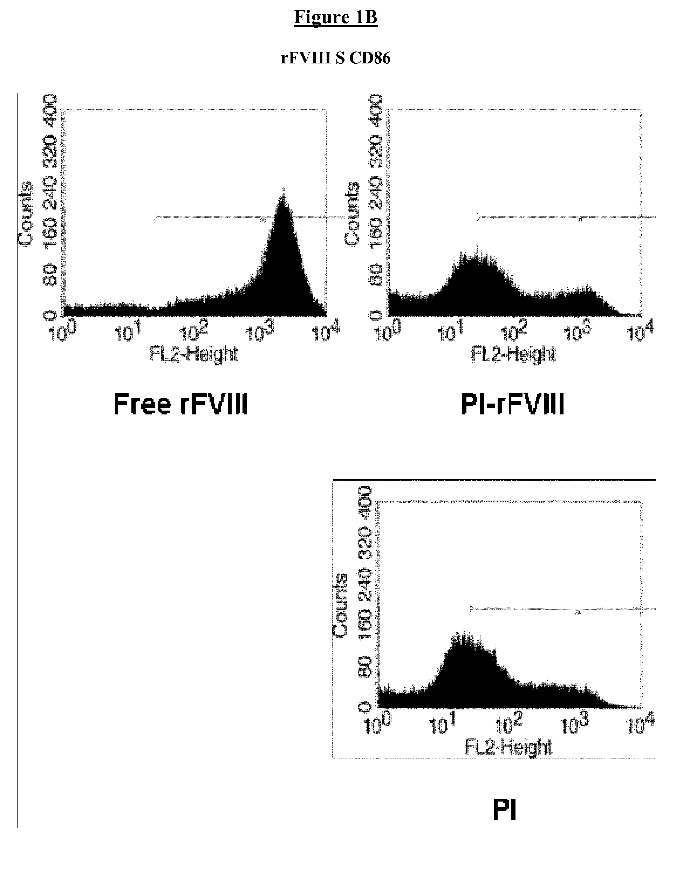

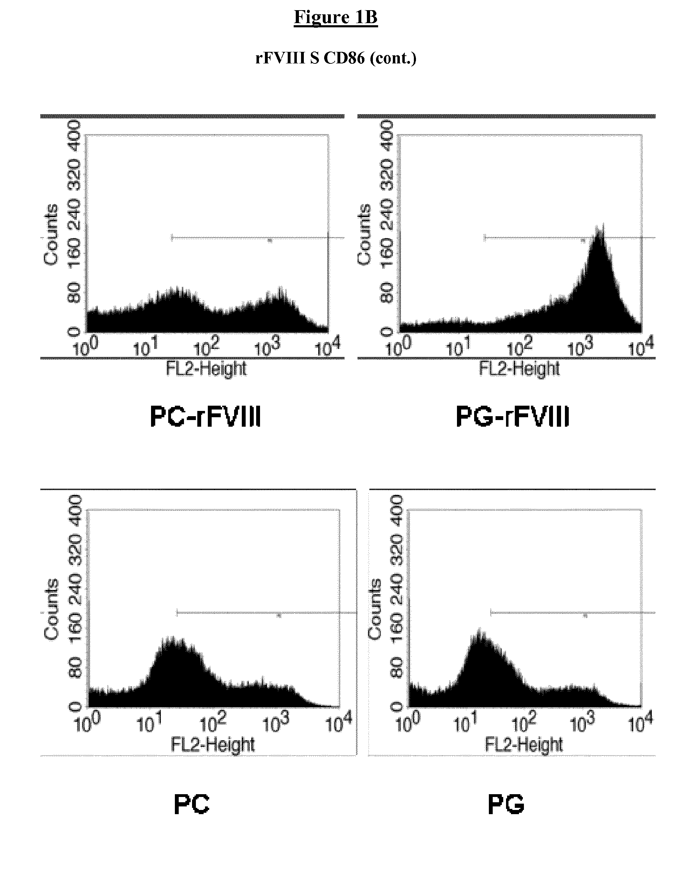

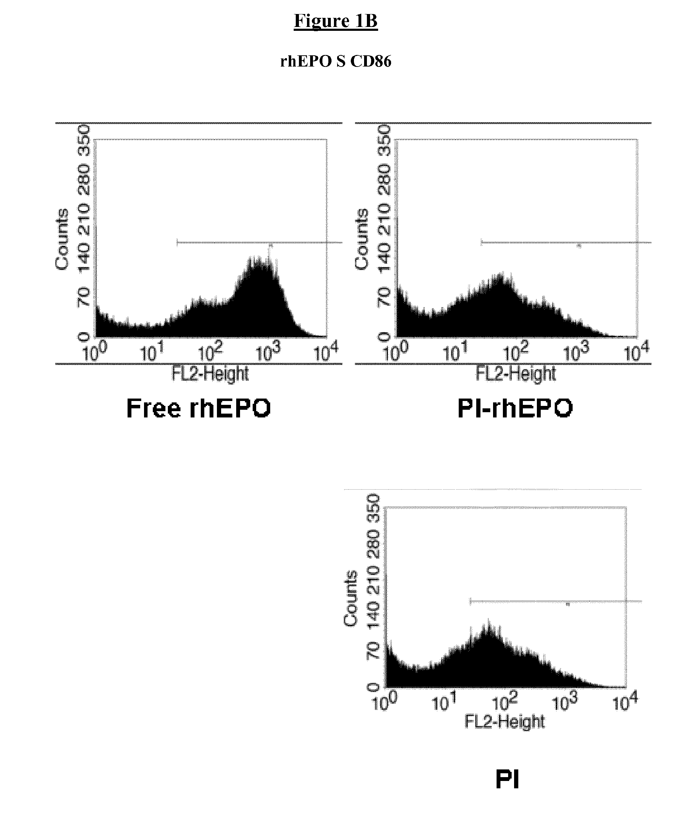

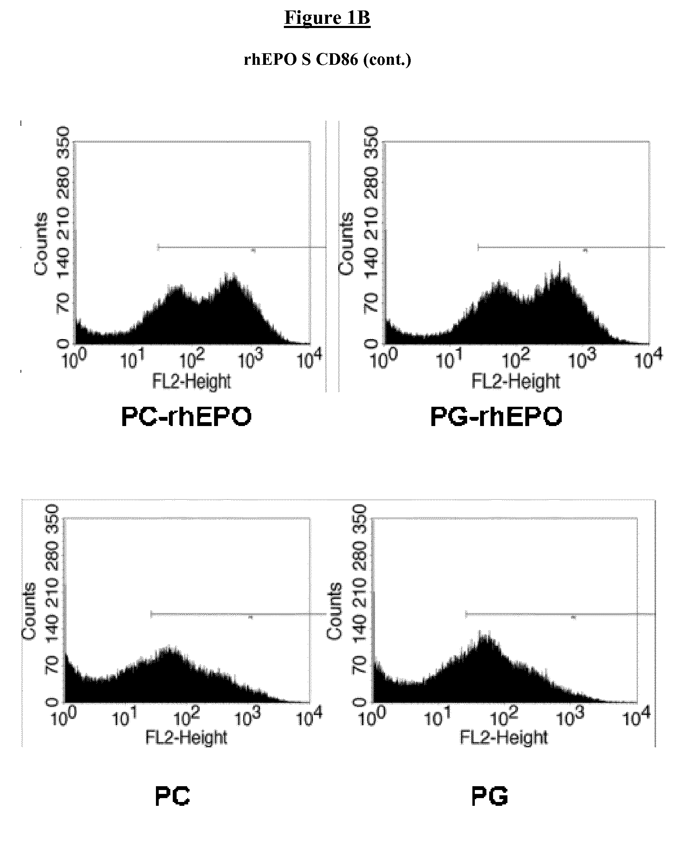

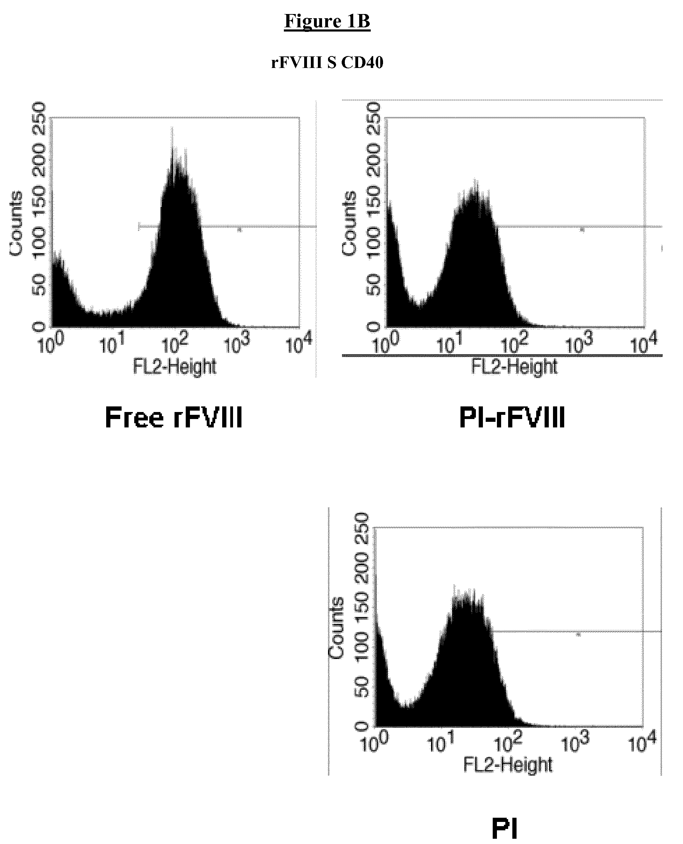

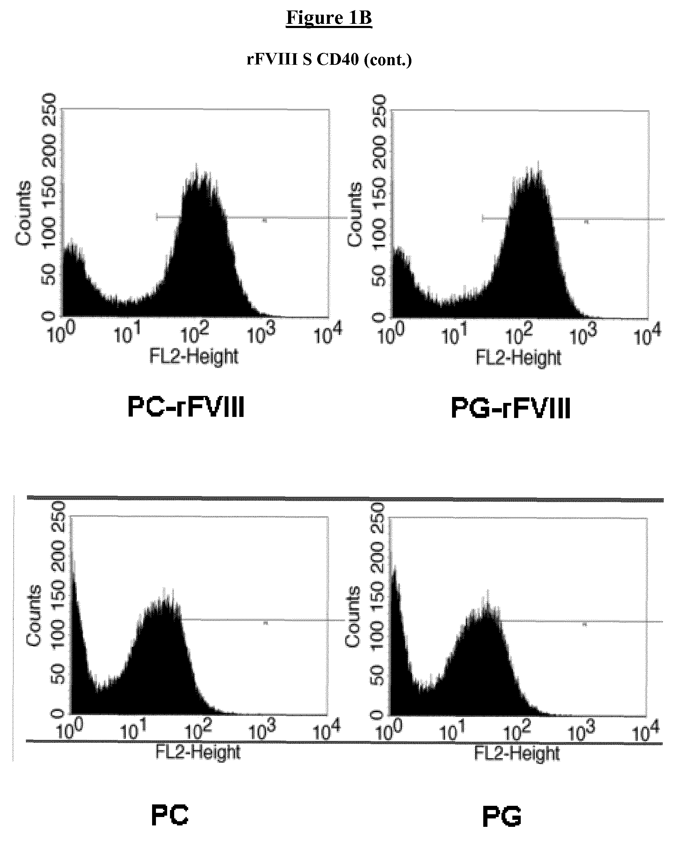

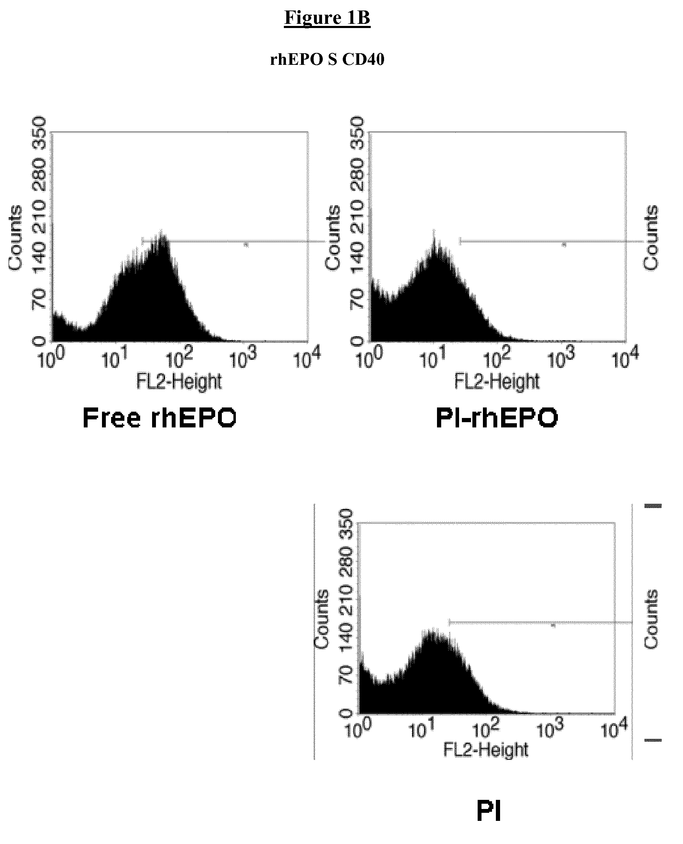

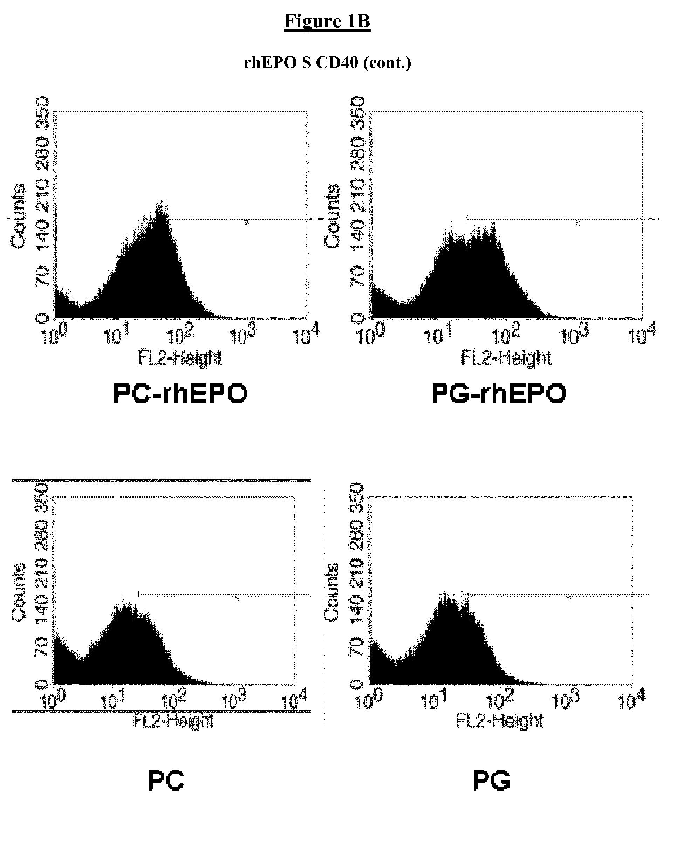

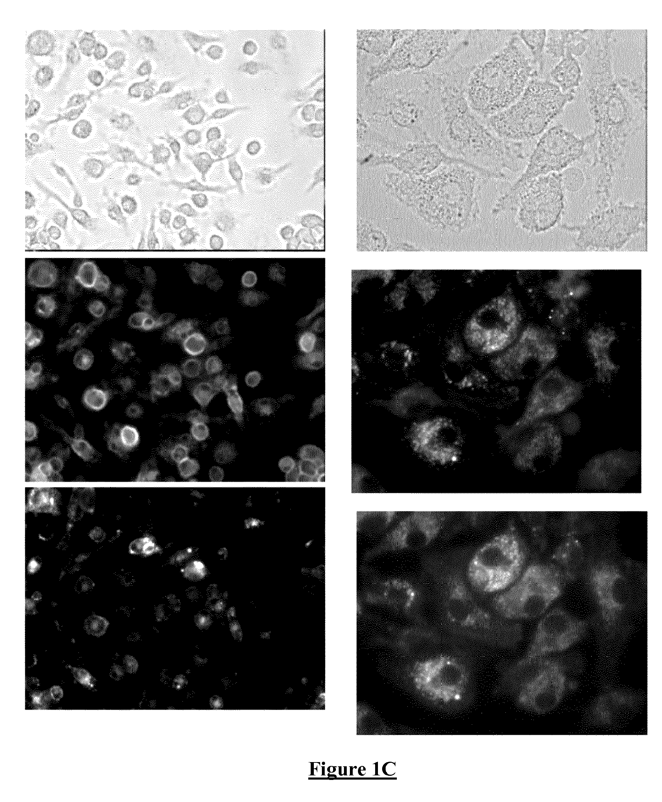

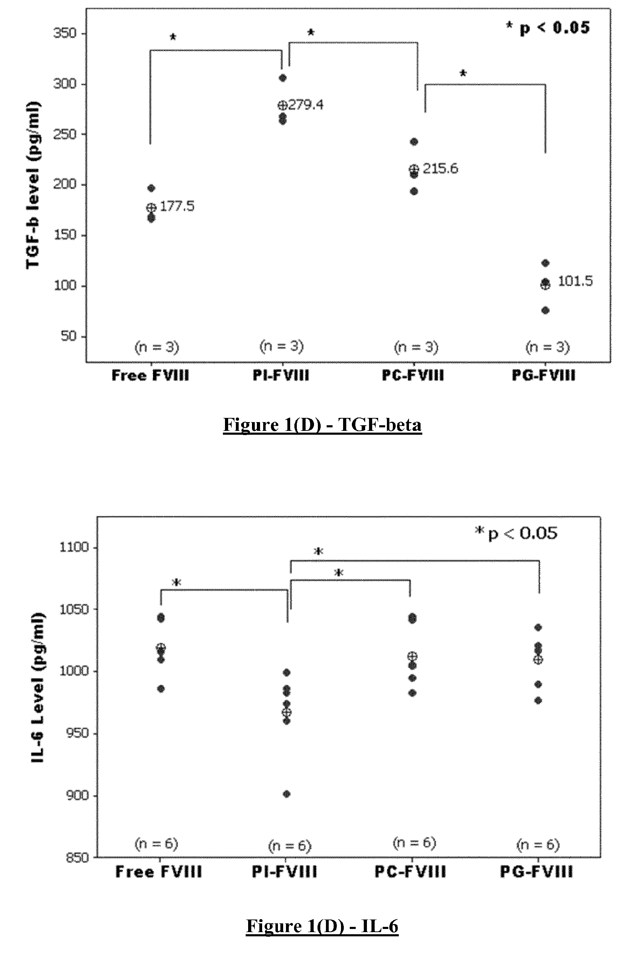

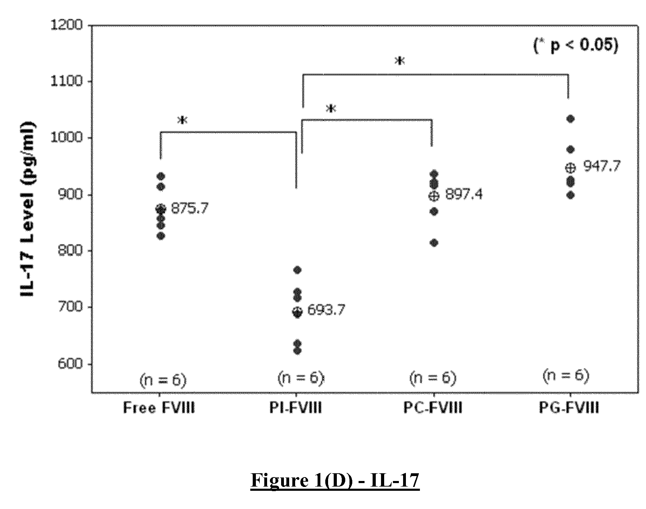

The results showed that association with PI nanoparticles reduced FVIII antibody responses in Hemophilia A mice (Figure 1A). Animals treated with rFVIII-PI complexes displayed significantly lower total antibody titers (Figure 1a and 1c) compared to animals treated with free rFVIII. For animals treated by s.c. injection, total anti-FVIII titers were 2379±556 (±S.E.M; n=10) for FVIII-PI, vs. 13,167 ± 2042 (n=15) for animals treated with free rFVIII. These differences were significant at P<0.05. For animals treated i.v. with rFVIII-PI, antibody titers were 3321±874 (n=8), compared to 4569±1021 (n=8) for animals treated with free rFVIII, and this difference was not significant. Interestingly, however, inhibitory antibody titers, which abrogate FVIII activity, were reduced significantly in animals given FVIII-PI by both s.c. and i.v. routes (Figure 1 b and 1d), compared to animals receiving free rFVIII. With s.c. administration, the inhibitory titers reduced by more than 70%. With i.v. administration, FVIII inhibitory titers were 675±71 for animals given free rFVIII, and were 385±84 for animals receiving rFVII-PI. This difference was statistically significant (p<0.05, one-way ANOVA, Dunnet's post-hoc analysis). Together, these results indicate that PI-containing lipidic nanoparticles not only reduced overall anti-rFVIII antibody titers, but, more importantly, lowered the titer of antibodies that abrogate the pharmacological activity of the protein.

In order to understand the immunological significance of the reduction in titers, in vitro studies aimed at understanding the mechanism of reduction in antibody response were carried out. The immune response against therapeutic proteins involve several steps that include presentation and processing of the protein by antigen presenting cells (APCs), presentation in the context of major histocompatibilty complex (MHC), interaction of APCs and T-cells (mediated by MHC II, T-cell receptor (TCR) interaction in the presence of co-stimulatory signals) followed by T-cell maturation, T-cell, B-cell interaction and B-cell maturation. The first step in this process is the antigen uptake and processing by antigen presenting cells. Since Dendritic cells (DCs) are important APCs involved in the immune response towards rFVIII, DCs isolated from naive hemophilic mice were used for immune response studies. Following treatment with FVIII, up regulation of cell surface markers MHC-II, CD11c, CD80, CD86 in immature Dendritic cells (iDC) were observed. The results indicate that FVIII act as an antigen and following its uptake, activation and maturation of DCs occur. The uptake and processing of FVIII in the presence and absence of lipid particles were followed to determine the effect of lipid structures on maturation of DCs (Figure 1B). FVIII in the presence of neutral lipid Phosphatidyl Choline (PC) and anionic lipid Phosphatidyl Glycerol (PG) containing liposomes were used as control and protein free lipid particles served as negative control. As is clear from the figure, an interesting observation is that PI lipid particles inhibited the up regulation of co-stimulatory markers, CD86 and CD40 following exposure of FVIII but control lipids, PC and PG did not interfere with the expression of co-stimulatory signals. The lipid alone, in the absence of FVIII had no or minimal observable effect on maturation of Dendritic cells. Similar observations were made when EPO was used as antigen (Figure 1B). Further, PG did not interfere with the up regulation of MHCII following uptake and presentation of FVIII and EPO whereas PI and PC reduced the expression of this phenotypic marker. In order to investigate the uptake of PI particles by DC, particles were labeled with fluorescent probe (HPTS) and the pH sensitive fluorescent properties of the probe was used to monitor endocytic uptake of the particles. The uptake of cationic liposomes containing N-[1-(2,3-Dioleoyloxy)Propyl]-N,N,Ntrimethyl ammonium methylsulfate (DOTAP) was used as a positive control due to high DC cellular uptake of cationic liposomes. After 30 min of incubation of 0.1 □mol of PI particle with DC, very little cell-associated fluorescence was observed (Figure 1C) but for other anionic liposomes PG and neutral liposomes containing PC, higher cell associated fluorescence was observed. Under similar experimental conditions, the cell-associated fluorescence for cationic lipid was much higher than all other lipid compositions. In illuminating conditions that excites pH sensitive fluorescence band of the probe, no change in fluorescence compared to total fluorescence was observed for DCs incubated with PI but substantial fluorescence intensity was observed for cationic liposomes. The data indicates that the endocytic uptake of PI particles is less compared to cationic liposomes and/or probably interferes with uptake and subsequent processing of FVIII and EPO leading to inhibition of the expression of co-stimulatory signals in DCs. The inefficient activation and maturation of the DC following treatment with protein can result in tolerizing rather than activating the T cells.

In order to determine whether FVIII specific T-cells were stimulated in vivo following immunization with FVIII-PI complexes, T-cell proliferation studies were carried out in vitro. The mean stimulation index (SI), which is the ratio of 3H-thymidine incorporation in the presence of antigen (100 ng/well) to incorporation in the absence of antigen, was significantly lower in the group treated with rFVIII-PI compared to those receiving free rFVIII, and this result was statistically significant (p<0.05).

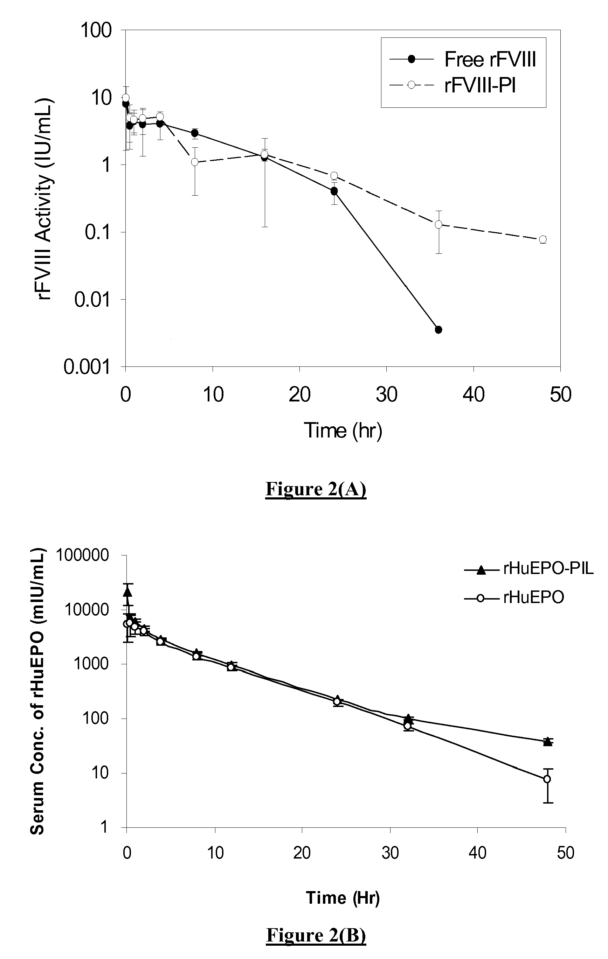

TGF-□□ is a regulatory cytokine that plays a critical role in regulating T-cell dependent immune responses. In order to determine whether TGF-□□ signaling contributes to the decrease in the observed antibody response to FVIII-PI, a cytokine analysis in vitro was carried out. CD4+ T-cells were isolated from rFVIII-immunized animals were co-cultured with Dendritic cells that were challenged with free rFVIII or a variety of rFVIII lipidic nanoparticle formulations. Cytokine secretion was quantified by ELISA (Figure. 1D). The results indicate a significant (p < 0.05) increase in the level of TGF-□□ upon exposure to rFVIII-PI complexes, relative to responses to free rFVIII or lipidic rFVIII formulations from which PI was omitted (ie., pure phosphatidylcholine), or in which the anionic phospholipid phosphatidylglycerol (PG) was substituted for PI (Figure. 2a). rFVIII-PI also increased the secretion of IL-10. IL-6 (Figure 2b) and IL-17 (Figure 2c) levels were significantly lower for the rFVIII-PI nanoparticle formulation, as compared to free rFVIII and the other lipidic rFVIII formulations (p < 0.05). The PI induced effect was observed when the antigen was changed from FVIII to EPO. EPO-PI increased the secretion of TGF-beta but reduced the secretion of IL-6 and IL-17 (data not shown). Previous studies have established that TGF-β1 induces the generation of T regulatory cells (Tregs) while TGF-β1 with IL-6 skews T cells into IL-17 producing TH17 cells. Based upon these findings our data are consistent with the possibility that FVIII-PI and EPO-PI may polarize the generation of naive T cells into Tregs by upregulating of TGF-β1 and suppressing of IL-6. This anticipated expansion of Tregs could explain the decreased T cell dependent anti-FVIII antibody response observed with FVIII-PI. Clearly the precise mechanism by which FVIII-PI reduces the antibody response remains to be established. It will be important to determine whether the decreased anti-FVIII antibody response is a reflection of Treg mediated specific immune suppression and/or immune tolerance that would result in a durable specific non-responsiveness to a subsequent exposure to FVIII. It will also be important to investigate the effect of such Treg mediated immune tolerance on the reduction of the antibody response that can break tolerance.

Pharmacokinetic studies were carried out in Hemophilia A mice to investigate whether PI nanoparticles prolong the circulation of FVIII. The data show that PI decreased the terminal slope (0.303 hr⁻¹ for FVIII and 0.0915 hr⁻¹ for FVIII-PI) and increased the terminal half-life of the protein (2.3 hrs for free FVIII and 7.6 hrs for FVIII-PI) (Figure 2A). An increase in Mean Residence Time (MRT) and Area Under the Curve (AUC) was observed. By the method of Bailer, the change in AUC did not appear to be significant.

As noted above, complexation with PI nanoparticles reduced significantly the terminal elimination rate of FVIII; this may be clinically relevant, in that the effect could increase the bleed-free time and reduce the frequency of administration. rFVIII activity was detectable 48 after administration of PI nanoparticle complexes, whereas replacement of PI with acidic phospholipid PS did not result in an extension of lifetime, and free FVIII activity was reproducibly detectable for only 24 hrs following administration. In order to investigate the therapeutic efficacy of FVIII-PI complexes and the potential clinical relevance of the observed increase in FVIII terminal half-life, a tail-clip model was employed. Because hemophilia A mice do not produce any active FVIII, survival and/or time to clot following the tail clip represent a pharmacodynamic endpoint of clinical relevance. However, tail clip survival assay in this animal model is not very accurate as substantial survival was observed even for buffer treated animals (Baru et al. Throm. Haemost 2005; 93;1061-1068) Therefore, time to clot was monitored as an indicator of efficacy. The tail clip was made in 3 animals 48 hrs after i.v. injection of rFVIII-PI and rFVIII a time at which levels of rFVIII were undetectable for the free-protein formulation. Clotting (bleeding stopped) was observed within 2 hrs for two thirds of the animals that were administered rFVIII-PI where as clotting was observed around 4 hrs for free rFVIII treated animal groups. The results indicate that the FVIII-PI complexes are efficacious. Furthermore, FVIII exposure (AUAC₀₋₂₄) was 64 IU.h/ml) following administration of PI nanoparticles, much higher than was observed for rFVIII complexed with PS liposomes (AUAC₀₋₂₄ 36 IU.h/ml). This finding is consistent with previous studies in which rapid uptake of PS liposomes by Kupffer cells of the RES was observed but PI resists binding to complement proteins and thus reduce its uptake by RES consistent with the observed "stealth-like" properties of PI. Pharmacokinetic studies suggest that PI particles containing other therapeutic proteins, such as erythropoietin alpha and Factor VIIa, also show higher AUCs, lower clearance, a shallower terminal slope, and altered biodistribution in appropriate animal models, compared to the free protein. The shallow terminal slope of PI- rHuEPO, λz (0.06 ± 0.01) hr⁻¹ is an indication of slow elimination phase that is in accordance with its lower apparent total clearance (CL) (9.49 ± 0.66) ml/hr (Figure 2B). CL for rHuEPO was equal to (12.69±1.31) ml/hr. The steeper terminal slope (0.13 ± 0.01) hr⁻¹ of rHuEPO implied that the protein is eliminated faster. Further, the AUC of rHuEPO-PIL was (47.89 ± 3.32) hr•mIU/mL and (36.16 ± 3.39) hr•IU/mL for the rHuEPO. The AUC showed that there was accumulation in the blood for protein bound to lipidic particles.

Thus, the results presented in this invention show that complexing a therapeutic protein with lipidic PI nanoparticles results in a multifunctional delivery strategy, in which protein complexation and physical stabilization are achieved, along with extension of pharmacokinetic properties, improvement of pharmacodynamic properties, and a beneficial immunomodulatory effect is exerted. This strategy is useful for number of therapeutic proteins (for example Factor VIII, EPO, Factor VIIa), and can improve safety and efficacy of protein based therapies.

### EXAMPLE 2

Exogenously administered recombinant FVIII (rFVIII) in the treatment of Hemophilia A has several problems including the development of inhibitory antibodies that abrogate the activity of the protein. It has been shown previously in our lab that rFVIII formulations containing Phosphatidylserine (PS) in particulate (PS-rFVIII) or in solution O-Phospho-L-Serine-rFVIII (OPLS-rFVIII) form reduces immunogenicity when administered in FVIII-knockout hemophilic mice. This example demonstrates the influence of PS-rFVIII and OPLS-rFVIII on T-cell clonal expansion, effect of PS on T-cell repertoire proliferation and the effect of PS on TGF-beta cytokine secretion. Dendritic Cells were isolated from bone marrow of naive hemophilic mice. The percentage of immature DCs (iDCs) was determined by flow-cytometry. T-cell proliferation study was done using CD4+ T-lymphocytes from spleens of hemophilic mice treated with various rFVIII formulations and challenging them with DCs incubated with free rFVIII. Similarly, proliferation of CD4+ T-cells upon injecting hemophilic mice with free rFVIII and iDCs incubated with different PS formulations was also studied. Proliferation was determined based on 3H-thymidine incorporation and expressed as stimulation index. Levels of TGF-beta cytokine in the culture supernatant were measured. Bone marrow from hemophilic mice was cultured to isolate DCs. MHC-II, CD11c, CD80, CD86 were chosen as the representative cell surface markers to characterize DCs and determine their maturation state. The maturation level of DCs was increased when they were pre-incubated with LPS. The proliferation of CD4+ T-lymphocyte clones was significantly lower (p< 0.05) in case of the PS-rFVIII and OPLS-rFVIII compared to animals treated with free rFVIII which was used as control. T-cell repertoire showed significant decrease in proliferation for PS-rFVIII compared to free rFVIII. There was significant increase in TGF-b level in OPLS-rFVIII compared to control. Further, the PS significantly reduced the seretion of IL-6 and Il-17. The data indicates that PS containing formulations suppresses the T-lymphocyte activity by Treg mechanism.

### Materials:

Full-length, purified and excipient-free rFVIII was obtained from Baxter Biosciences (Carlsband, CA). The stock solution of the recombinant protein used to prepare the samples had a specific activity of 2466 IU (∼0.5 mg/ml). Normal (control) plasma, FVIII-deficient plasma and Platelin L aPTT assay reagents were purchased from Trinity Biotech (Co Wicklow, Ireland). Brain phosphatidylserine (BPS) and Dimyristoylphosphatidylcholine (DMPC) were obtained from Avanti Polar Lipids (Alabaster, AL), stored in chloroform at -80°C, and used without further purification. Sterile, pyrogen free water and Isoflurane were purchased from Henry Schein Inc. (Melville, NY). The monoclonal antibody ESH 8 was purchased from American Diagnostica Inc. (Greenwich, CT). O-phospho-L-serine (OPLS), phosphocholine (PGhg), IgG-free bovine serum albumin (BSA), sodium pyruvate, dextran, Imidazole, Tween 20, Potassium Iodide, Phosphocholine chloride calcium salt, Cholesterol, Hydrogen peroxide and standard phosphorus solution were obtained from Sigma (Saint Louis, MO). p-Nitrophenyl phosphate disodium salt was purchased from Pierce (Rockford, IL). Dynal CD4+ negative isolation kit, RPMI-1640 culture medium, penicillin, streptomycin, L-Glutamine, 2- mercaptoethanol and Polymyxin B were all obtained from Invitrogen Corp., (Carlsband, CA). ³H-thymidine and Unifilter 96-well plate were obtained from Perkin Elmer Inc. (Boston, MA). Maxisorp 96-well ELISA plates and 6-well flat-bottom sterile plates were purchased from NUNC. TGF-beta development kit was obtained from R&D systems. Syringes, needles and cell-strainer were obtained from Becton. rmGMCSF was obtained from Peprotech and Fetal Bovine Serum was from Biowhittaker. All other buffer salts used in the study were purchased from Fisher Scientific (Fairlawn, NJ).

### Methods:

### Isolation of dendritic cells:

Dendritic cells were isolated from the bone marrow of hemophilic mice. A naïve hemophilic mouse was anesthetized using isoflurane inhalation. To maintain a clean environment and minimize contamination, autoclaved scissors and forceps were used and the mouse was disinfected with 70% alcohol. The mouse was sacrificed by cardiac punctured. The hind limbs which include the femurs and tibiae were obtained and any attached muscle or skin was removed carefully so as to not break the bones. The bones were placed in 70% alcohol for disinfection. The bones were later placed in a sterile petri-plate in a cell culture hood. The ends of the bones were cut and ice-cold, sterile and iso-osmotic PBS at pH 7.0 was flushed with the help of a 10ml sterile syringe with a 25 gauge needle. The flushed bone marrow was collected in the Petri plate. The cells were pipetted gently and passed through a cell strainer to loosen any cell clumps as well as to remove debris. The cell suspension was centrifuged at 300g for 10 minutes at 4°C. The supernatant was discarded and the cells were resuspended in residual medium. Viable cells were counted under a microscope using the hemocytometer and trypan-blue method. Accordingly, 2 x 10⁶ cells were plated in a sterile petri-plate along with 1ml of sterile FBS, 4uL of 200units/ml of rmGMCSF and RPMI-1640 media containing Penicillin-Streptomycin, 2-mercaptoethanol and L-Glutamine, making the total volume up to 10ml. This was considered as day '0'. The cells were incubated at 37°C and 5% CO2. On day 3, another 10ml of media containing rmGMCSF and FBS was added to the petri-plate. On day 6 and 8, 10ml of the supernatant from the petri-plate was aspirated and centrifuged in a 15ml centrifuge tube at 300g for 10 minutes at 4°C. Supernatant was discarded and the cell pellet was resuspended in 10ml of fresh medium containing rmGMCSF and FBS. On day 10, cells were harvested by gentle pipetting. The cells thus obtained were further used for characterization and other experimental studies.

### Characterization of Dendritic Cells:

In order to verify the differentiation of bone marrow cells into dendritic cells, characterization of the cell's surface markers' expression level was performed. This was determined by using fluorescently-tagged antibodies for specific cell surface marker of interest and analyzing the antibody-tagged cells using flow cytometry. The cell surface markers which were identified as important in this study were MHC II, CD11c, CD40, CD80, and CD86. Briefly, the harvested cells on day 10 were washed two times with ice cold sterile PBS. Viable cells were counted as described earlier. The concentration of the viable cells was adjusted to 1 x 10⁶ cells/ml. Accordingly, 1ml of the cells was added to 10 different flow cytometry tubes and another 3ml of ice cold PBS was added to each of the tubes. The tubes were centrifuged at 1000g for 3-5 minutes at 4°C. Supernatant was discarded and the tubes were inverted and blotted. The cell pellet was resuspended in residual (∼100uL) volume. Fcblock antibody was added to all tubes and incubated for 10 minutes on ice. An appropriate volume of different fluorescently-tagged antibodies was added to the corresponding tubes and incubated for 15 minutes on ice in dark. Appropriate isotype antibody controls were also included in the study. 4ml of ice cold PBS was added to each of the tubes and centrifuged at 1000g for 3-5 minutes at 4°C. The supernatant was discarded and 0.5 ml of 2% ultrapure paraformaldehyde was added to fix the cells and the tubes were covered with aluminium foil to prevent exposure to light and were later analyzed using a flow cytometer.

### Determination of immature dendritic cells:

To determine the percent of iDCs present in the culture medium, the harvested dendritic cells were stimulated with Lipopolysaccharide (LPS). LPS is a well known strong antigen. The rationale was that if there are naive iDCs present in the medium, they will take up LPS and present its processed antigenic fragments on their surface. Consequently, there will be change in the expression level of the cell surface markers. If the DCs have already matured before the addition of LPS due to other experimental factors, they will not take up LPS since mature DCs have lost their phagocytic capacity. Measuring the shift in peak in flow cytometry analysis before and after incubating with LPS, the percent of iDCs was determined. On day 9 of cell culture, two groups of dendritic cells were selected. One group was used as a negative control while the other group was incubated with LPS. Both the groups of cells were incubated at 37°C and 5% CO2 for 24 hrs (Day 10) and the cells were prepared for flow cytometry as described earlier.

### Preparation of the different formulations:

**BPS-liposomes:** BPS and DMPC in chloroform were mixed in a 70:30 molar ratio and the chloroform was evaporated using a rotary evaporator to form a thin lipid film. The film was rehydrated using sterile Ca+2-containing Tris buffer at pH 7.0 and the solution was extruded multiple times using a Lipex extruder having a 200nm pore size membrane. After particle sizing and achieving the required particle size range, the liposomes were sterile filtered using a 0.22um syringe filter. Phosphate assay was performed to calculate the lipid recovery. Accordingly, rFVIII was associated with the liposomes in a 1: 10,000 protein: liposomes molar ratio and incubated at 37°C for 30mins for the association.

**PC and PG liposomes:** Control liposomes of PC (DMPC) (PC:cholesterol, 100:5 mol ratio) and phosphatidylglycerol (DMPG) (PG) (PG:PC:cholesterol, 50:50:5 mol ratio) were prepared using the same procedure as for PS liposomes. The particles comprising only PC as the phospholipid are referred to herein as PC liposomes, PC particles, PC lipidic particles or PC nanoparticles. The particles comprising PG and PC are referred to herein as PG liposomes, PG particles, PG lipidic particles or PG nanoparticles.

**OPLS solution:** 10mM OPLS solution was prepared in Ca-Tris buffer using pyrogen-free water. The pH was adjusted to 7.0 and the solution was sterile filtered through 0.22um syringe filter. Required amount of rFVIII was dissolved in the solution to have a 2ug/100ul (injection volume) rFVIII concentration.

**PChg solution:** 10mM phosphocholine solution was prepared in pyrogen-free Ca-Tris buffer. The pH was adjusted to 7.0 if necessary and the solution was sterile filtered through a 0.22 um syringe filter. A rFVIII concentration of 2ug/100ul was prepared accordingly.

### Development of Total & Inhibitory antibodies:

In order to observe the comparison between antibody titers in hemophilic mice upon administration of different formulations, three groups of naive male hemophilic mice were selected. The three treatments were free rFVIII, OPLS-rFVIII, PChg-rFVIII. Six animals were used per treatment group and each animal received 2ug of rFVIII i.v. in the respective formulation (100ul) once a week for four consecutive weeks via the penile vein. Blood was collected at the end of 6 weeks by cardiac puncture and centrifuged at 5000 rpm for 5 min at 4°C. Plasma was collected carefully and stored at -80°C until further analysis.

### Total antibody titer determination:

Total antibody titers were measured by using an ELISA technique with ESH8 antibody being the standard. ELISA plate (maxisorp, NUNC) was coated with 50ul of 2.5ug/ml of sterile rFVIII in sodium carbonate buffer (0.2 M) pH = 9.6. The plate was incubated overnight at 4°C. The plate was washed six times with PBS containing 0.05% Tween 20 (PBS-T, 250ug/well) using an automated plate washer. The plate was blocked with PBS containing 1% BSA (200ul/well) as the block buffer for 2hr at room temperature.. The plate was washed six times with PBS-T. Detection antibody (1:1000 dilution and 50ul/well) in block buffer was added to the wells and incubated for 1 hr at room temperature. The plate was washed six times with PBS-T. 1mg/ml of PNPP solution (100ul/well) was added to the wells and incubated for 30min at room temperature. The reaction was read immediately at 405 nm using a micro-plate reader. Based on the ESH8 standard curve, the total antibody titers for the plasma samples were determined.

### Determination of inhibitory anti-rFVIII antibody titers:

To determine the inhibitory antibody titers, FVIII-deficient human plasma, normal pooled human plasma and APTT-L reagent kit was acquired from Trinity Biotech. Reconstitution was done as per the manufacturer's instructions. Serial dilutions of normal plasma were done using imidazole buffer of pH 7.4. aPTT assay was performed with 100ul of normal plasma and 100ul of rFVIII-deficient plasma and later adding the APTT-L reagents and measuring the clotting time. Mouse plasma samples were also diluted using FVIII-deficient plasma. Each dilution of the plasma sample was mixed with equal volume of normal plasma and incubated for 2 hr at 37°C and aPTT assay was performed to measure the clotting time. Based on the standard curve, the inhibitory titers were determined.

### T-cell clonal expansion:

Upon interaction with mature dendritic cells in the lymph node and based on the type of signal, T-lymphocytes either undergo a clonal expansion or the expansion is arrested. Generally, upon identification of known antigenic fragments, T-cells undergo clonal expansion. The rationale for this study was to determine if the lipidic formulation had any role in influencing the T-cell clonal expansion. Five groups of ≥6 animals each were selected. The five groups were injected s.c. with 2ug of rFVIII in different formulations viz. plain Tris buffer (free rFVIII), BPS-liposomes, DMPC-liposomes, OPLS solution and PChg solution once weekly for two consecutive weeks. Simultaneously, on the day of week one injection, a naive hemophilic mouse was sacrificed and bone marrow was collected for dendritic cell culturing as described earlier. On day 9 of DC culturing, the cells were incubated with 2ug/ml of free-rFVIII. Three days after the second injection, spleens were collected and ground using a pestle in the cell-culture hood and passed through a cell-strainer. The cells were then centrifuged at 200g for 10mins at 4°C. The supernatant was discarded and the cell pellet was resuspended in 3ml of buffer 1 prepared as per the CD4+ negative isolation kit manual. The total T-lymphocyte count was determined by cell-dyne instrument. Accordingly, 3x10⁷ cells were used for the negative isolation assay. The assay was performed as per the steps mentioned in the isolation kit manual. After isolation, the number of CD4+ T-lymphocytes was determined by cell-dyne. The day 10 DCs which were incubated with free rFVIII for 24hrs were harvested, thoroughly washed and viable cells counted. Later, 2 x 10⁵ CD4+ T-lymphocytes were plated along with 7x10⁴ DCs in quadruplicate in T-cell proliferation media in two 96-well tissue culture plates. Only T-lymphocytes without DCs served as the negative control whereas T-lymphocytes alongwith naive DCs (who were not exposed to rFVIII) and Concanavalin A (Con A) served as the positive control. The cells were incubated for 72 hr at 37°C and 5%CO2. One plate was used for cytokine analysis and the second plate for T-cell proliferation study. After 72 hrs, one plate was centrifuged at 300g for 10min at 4°C and the supernatant was carefully collected. The sample was stored at -80°C until further cytokine analysis. To the second plate, 1uCi/well of ³H-Thymidine was added and incubated for another 16 hr. At the end of the 16hrs, the plate was harvested using a harvester on a Uni-filter plate and the counts per minute (cpm) of ³H-Thymidine were measured using a scintillation counter. Stimulation Index was calculated as the ratio of average count of samples to the average count of the negative control.

### Cytokine analysis:

The stored sample supernatants were analyzed for the level of Transforming Growth Factor-beta (TFG-b) using a developing ELISA kit for TGF-b detection from R&D systems. The procedure was followed as per the manufacturer's instructions.

### T-cell repertoire study:

After looking at the influence of lipidic formulations on T-cell clonal expansion, the next objective was to study the uptake by DCs of the different formulations and how similar or differently the processing and presentation happens for these different rFVIII formulations. The rationale for this study was to have the T-cells exposed to free rFVIII in vivo and later subjecting these T-cells to cultured DCs who earlier have been incubated with different rFVIII formulations in vitro. The study was to observe any influence of the lipidic formulation on the rFVIII uptake & processing by DCs. Five groups of three naive hemophilic mice were selected. The groups were injected s.c. one weekly for two consecutive weeks with 2ug free rFVIII. Simultaneously, a naive hemophilic mouse was sacrificed and DCs were cultured. On day 9 of DC culture, five groups of DCs were incubated separately with five different formulations viz. free rFVIII, liposomal-BPS-rFVIII, liposomal-DMPC-rFVIII, OPLS-rFVIII and PChg-rFVIII for 24hrs. Three days after the second injection, the animals were sacrificed and their spleens collected & processed as described earlier. Accordingly, 2x10⁵ CD4+ T-lymphocytes were incubated with the DCs who had been incubated with the five different formulations. Only T-cells in medium served as the negative control whereas T-cells incubated with naive DCs (which were not exposed to rFVIII) and ConA served as positive control. Further cytokine analysis and proliferation was done as mentioned earlier.

### Results and Discussion:

### Percent maturation of DCs:

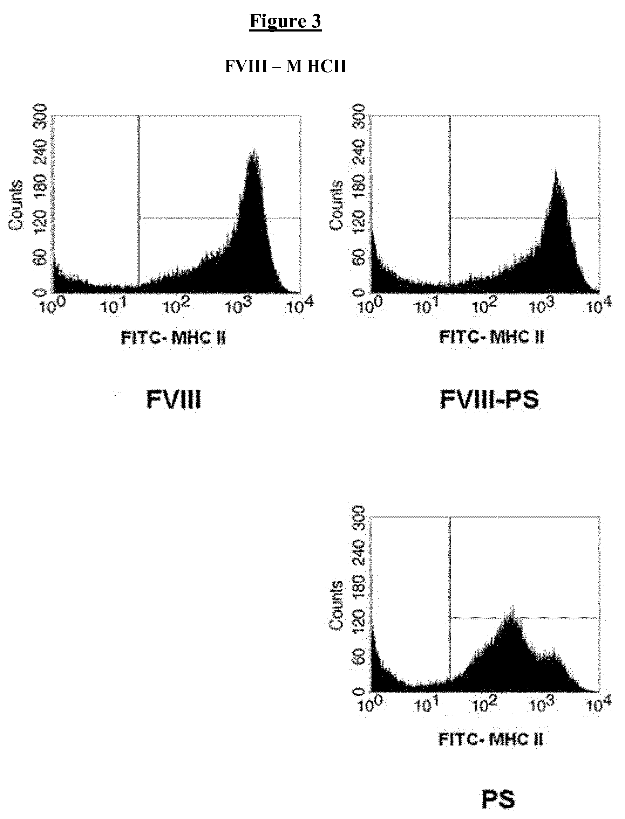

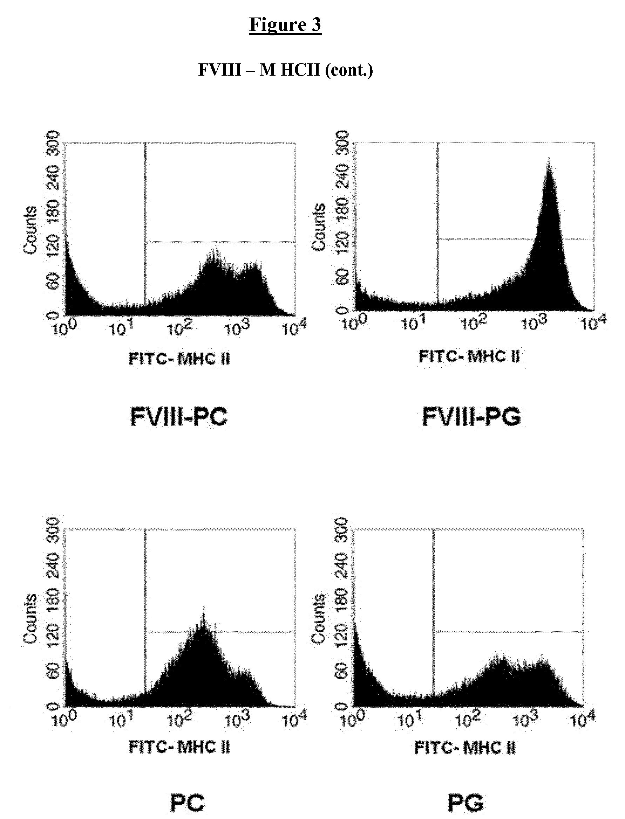

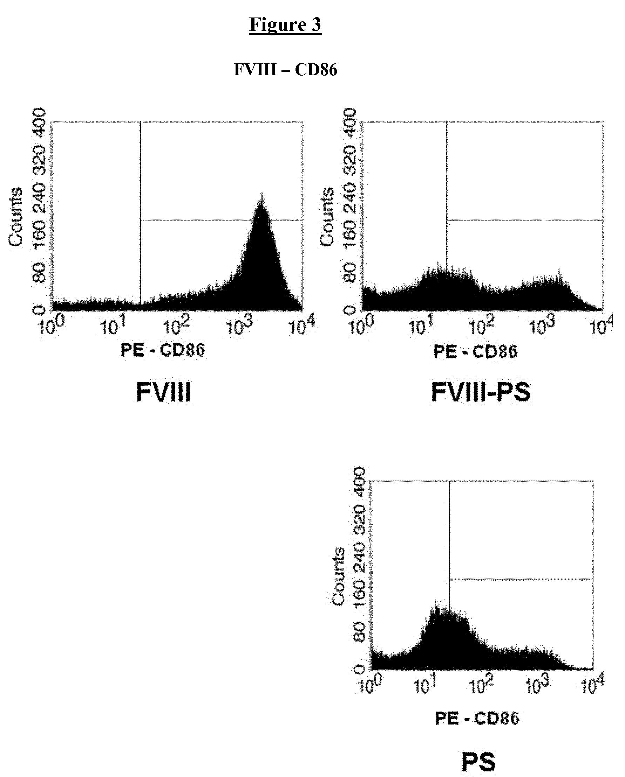

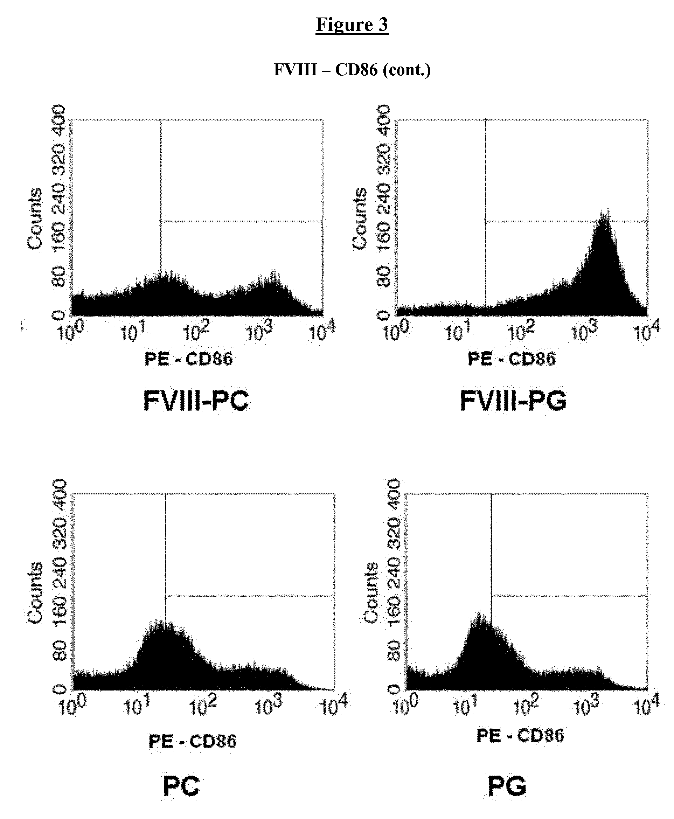

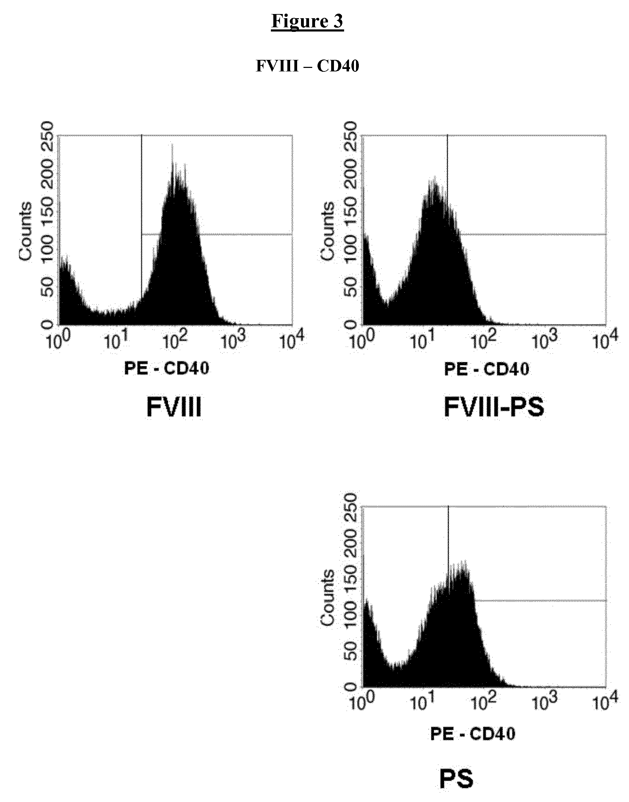

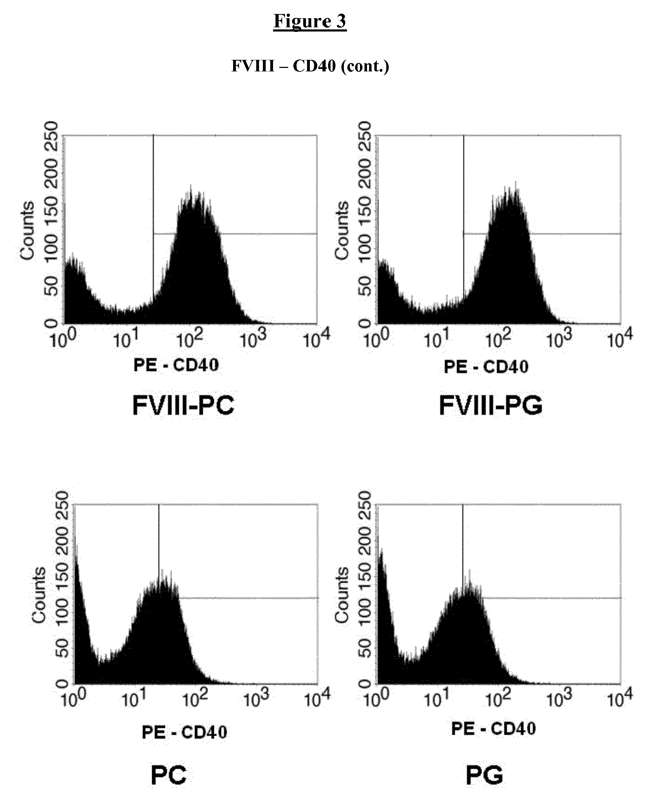

The uptake and processing of proteins by APCs was examined as it constitutes the first step in the immune response towards therapeutic proteins. Because immunogenicity against FVIII is a T-cell dependent process and dendritic cells are the principal initiators of T-cell responses, the maturation and activation of DCs was investigated in response to FVIII alone or complexed with PS-containing liposomes (Fig.3 A-C). Phenotypic maturation was followed using MHC II, CD86 and CD40 as the surface markers. When DCs were exposed to free FVIII, an increase was observed in all phenotypic markers. Upon exposure to FVIII-PS complexes, the increase in MHC II expression (Fig. 3A) was similar to that observed with exposure to free FVIII, suggesting that PS did not interfere with FVIII-mediated maturation of DCs. However, PS reduced the up-regulation of the co-stimulatory molecules, CD86 and CD40 (Fig. 3B & 3C respectively). This lipid-mediated effect was specific for PS liposomes, and was not observed with liposomes of similar electrostatic charge in which PG was substituted for PS. However, DCs exposed to PC liposomes associated with FVIII showed lower MHC-II and CD86 expression compared to cells exposed to free FVIII, but the up-regulation of CD40 expression was not inhibited. Thus the data suggest consistent PSmediated reduction in the up-regulation of both co-stimulatory signals upon FVIII exposure. Similar PS-mediated inhibition of co-stimulatory markers was observed for human dendritic cells and for bone marrow-derived mouse dendritic cells in response to LPS stimulation.

### Total Antibody Titers:

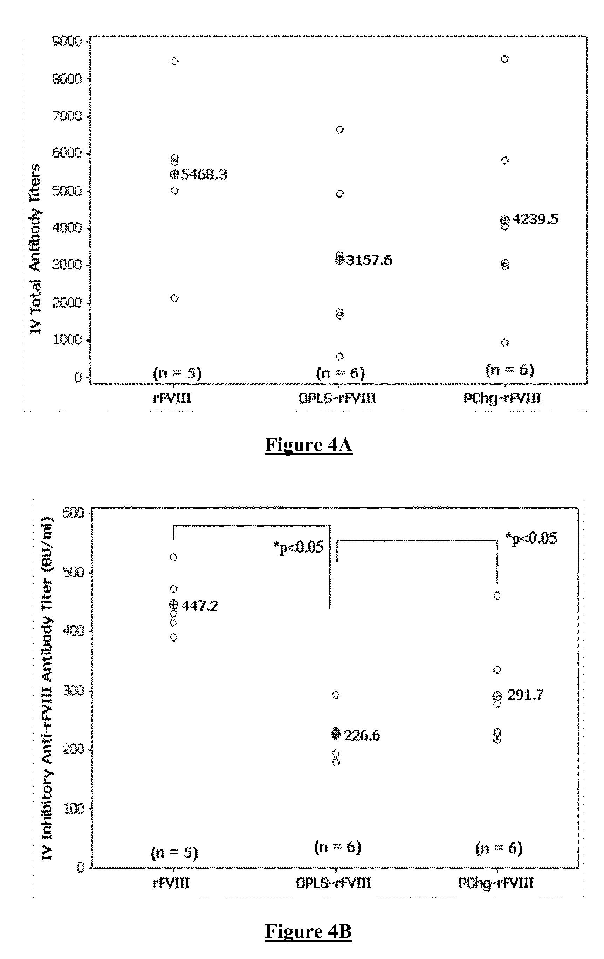

The total antibody titers for BPS-liposomal formulation was found to be lower compared to free rFVIII. In this study, the effect of solution-state phosphatidylserine containing formulation (OPLS) on the development of total antibody titers was performed (Figure 4A). Phosphocholine (PChg) containing formulation was used as control. Based on ELISA, the total Antibody titers for OPLS-rFVIII (3157, n = 6) were lower compared to free rFVIII (5468, n = 5) and PChg-rFVIII (4239, n = 6). Due to high variability in the data a statistical significance could not be established.

### Inhibitory anti-rFVIII Antibody Titers:

Clinically, inhibitory anti-rFVIII antibodies have more relevance. The inhibitory antibodies abrogate the activity of a therapeutic protein thus, causing a loss or decrease in its overall efficacy. Generally, higher level of inhibitory antibodies against a therapeutic protein indicates a higher level of immunogenicity being observed in the patient towards that particular therapeutic protein. Here, the titer of inhibitory antibodies was measured using a one-stage aPTT clotting assay. Based on this assay, the inhibitory anti-rFVIII antibody titers for OPLS-rFVIII (226.6, n = 6) were significantly lower (p < 0.05) compared to free rFVIII (447.2, n = 5) as well as PChg-rFVIII (291.7, n = 6). See Figure 4B. The statistical analysis was done by one-way ANOVA. This result suggests that OPLS may have some protective property.

### T-cell clonal expansion:

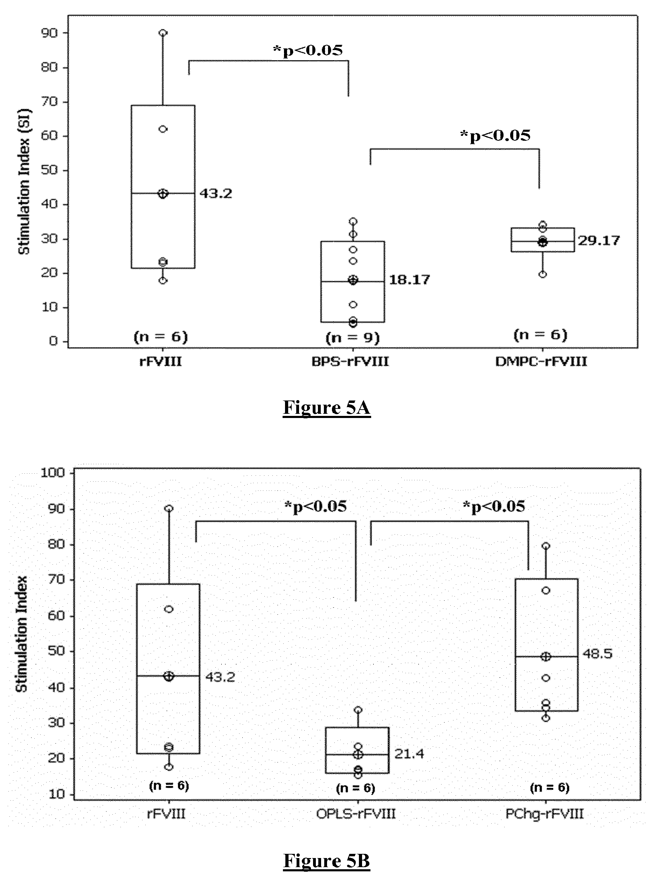

T-cells are highly specific and are activated only if the right antigenic fragment(s) is presented to them. It also depends on the co-stimulatory signals being sent by the antigen presenting cell which can activate or suppress the activation of T-cells. Suppression will lead to lower proliferation. The influence of the lipidic nature of the formulation on the T-cell proliferation was studied. T-cells that were exposed to the liposomal-BPS-rFVIII formulation group showed a significant decreased proliferation (18.17, n = 9, p<0.05) compared to the free rFVIII (43.2; n = 6) and the liposomal-DMPC-rFVIII group (29.17; n = 6; p<0.05) (Figure 5A). Similarly, the solution state OPLS-rFVIII group showed significantly reduced proliferation (21.4; n = 6; p<0.05) compared to free rFVIII (43.2; n =6) as well as PChg-rFVIII (48.5; n = 6; p<0.05). See Figure 5B. The results support that phosphatidyserine containing formulations have an suppressive effect on the T-cell proliferation.

### T-cell repertoire study:

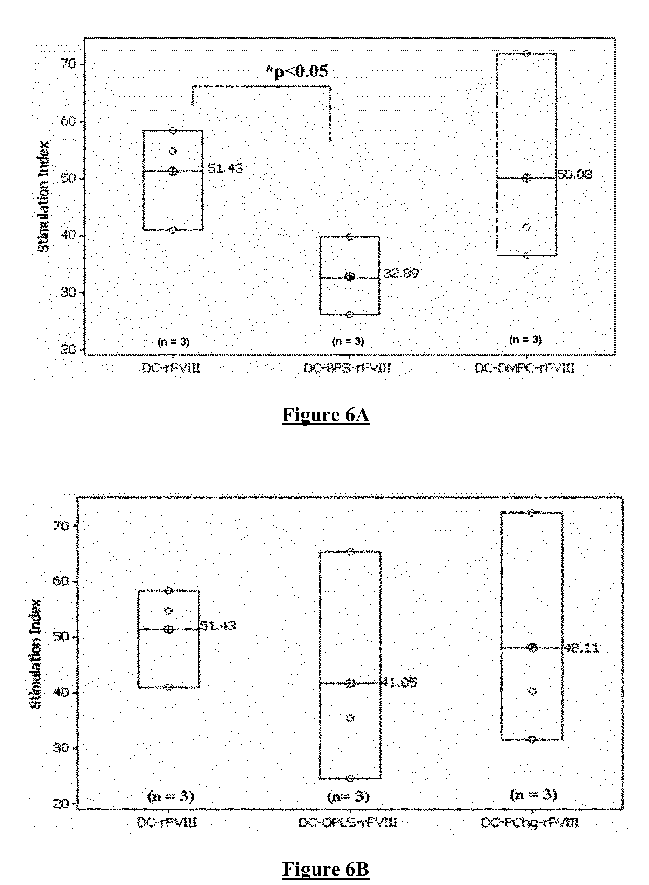

To understand the lipidic formulations' role on the uptake by iDCs, different lipidic formulations were incubated with DCs and later co-cultured with T-cells obtained from spleens of animals injected with free rFVIII. The T-cell proliferation was significantly reduced for the liposomal BPS-rFVIII formulation group (32.89; n = 3) compared to the free rFVIII (51.43; n = 3) and reduced compared to liposomal DMPC-rFVIII (50.08; n = 3; p>0.05) (Figure 6A). Although a statistical significance could not be established, there was a reduction in proliferation observed for the OPLS-rFVIII group (41.85; n = 3) as compared to free rFVIII (51.43; n = 3) and PChg rFVIII (48.11; n = 3) (Figure 6B). This suggests that the phosphatidylserine containing formulations interfere with the uptake of rFVIII by dendritic cells. This could lead to low or misprocessing of the rFVIII eventually leading to the observed lower T-cell proliferation.

### Cytokine Analysis:

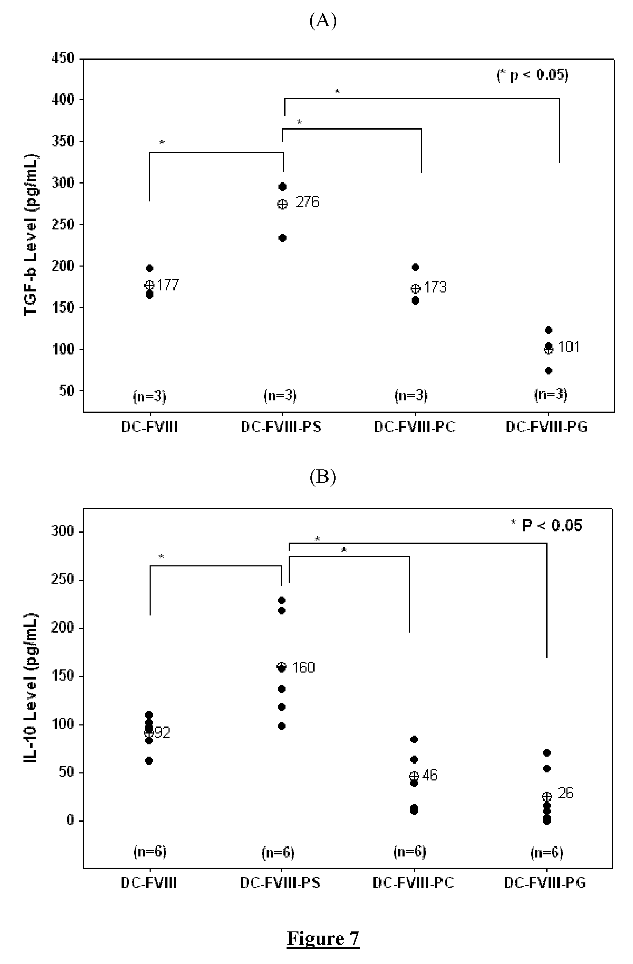

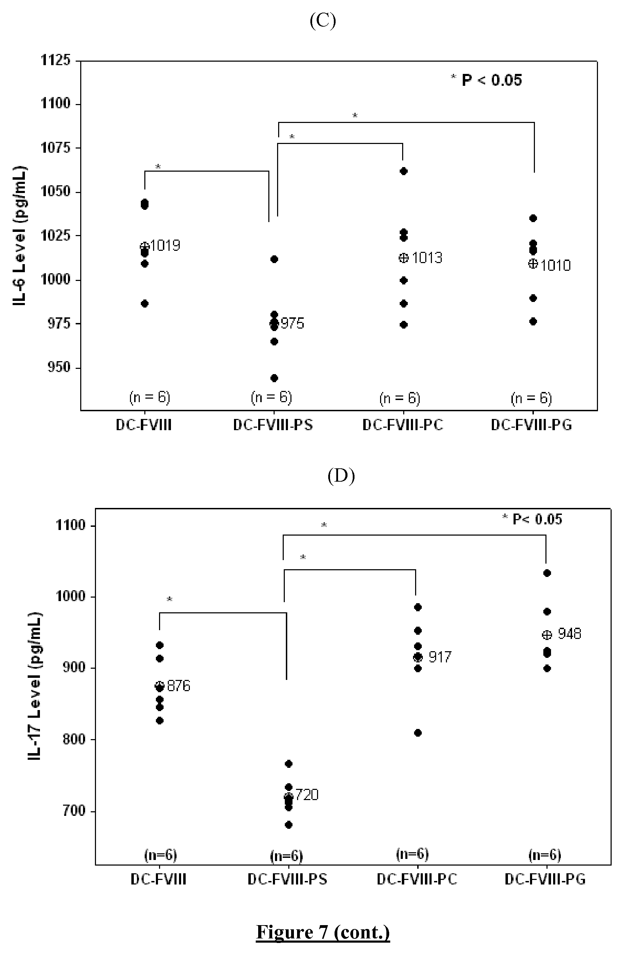

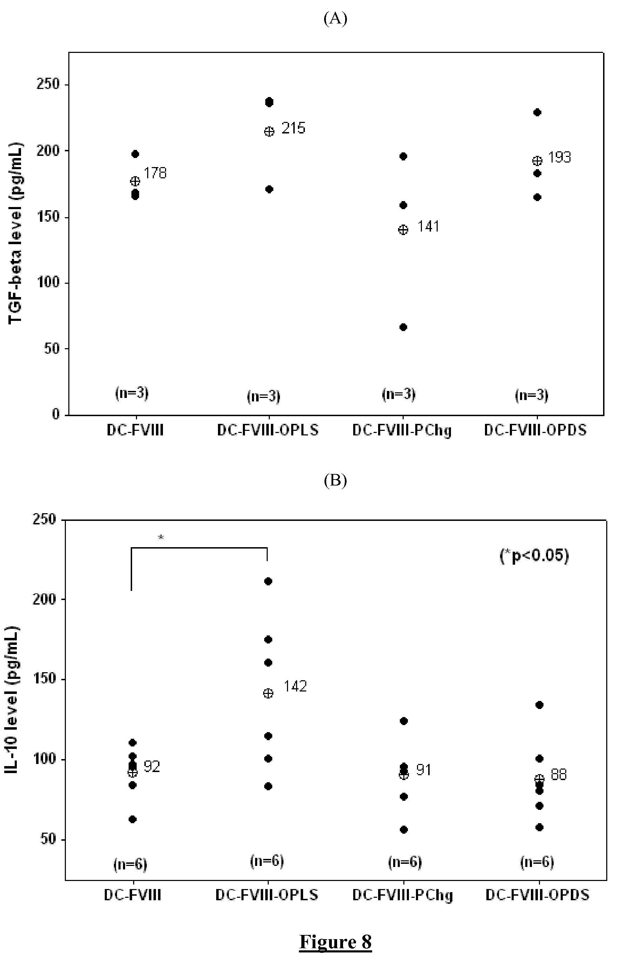

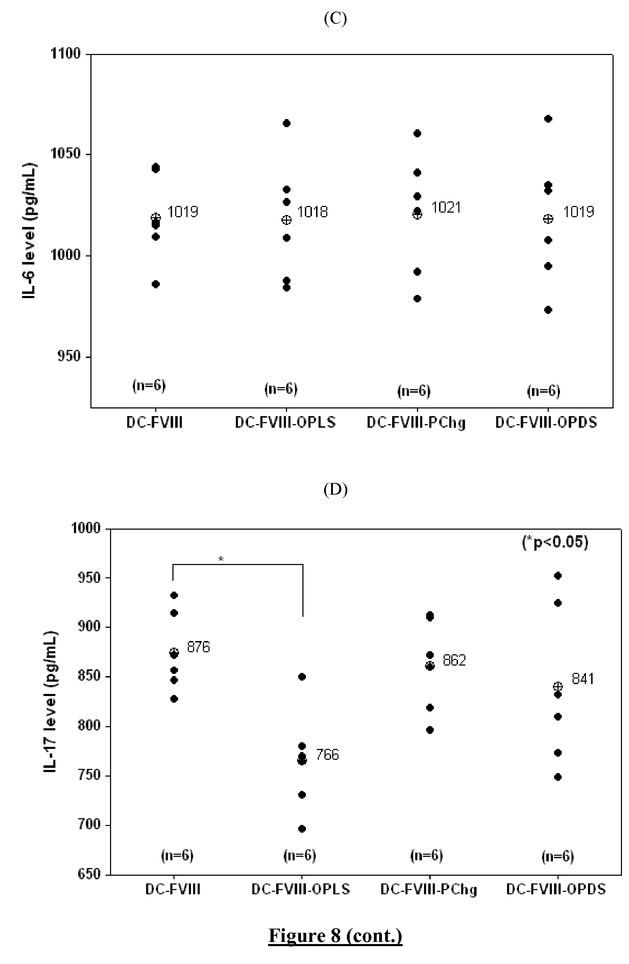

In addition to the epitope presentation, it is also very important to study the co-stimulatory signals as they also have an effect on the T-cells. One of the co-stimulatory signals is the secretion of various cytokines by the antigen presenting cell. The type and level of cytokine secretion depends on whether a proliferation or suppression is intended. Transforming Growth Factor-beta (TGF-b) is one such cytokine that is known to have significant importance. It is believed that TGF-b has an anti-proliferative effect on the T-cells. There was an increase in the TGF-b levels in the liposomal BPS-rFVIII group (217.35; n = 6) as compared to the free rFVIII (155.06; n = 6) and liposomal DMPC-rFVIII (170.12; n = 6) (Figure 7). Similarly, the OPLS-rFVIII group showed a significantly higher TGF-b levels (223.28; n = 6; p<0.05) compared to free rFVIII (155.06; n = 6) and PChg-rFVIII (147.31; n = 6; p<0.05) (Figure 8). So, the suppression of T-cell proliferation may be in part due to the higher levels of TGF-b being secreted.

While this method and composition has been shown and described with reference to certain preferred embodiments thereof, it will be understood by those skilled in the art that various changes in form and details may be made therein without departing from the spirit and scope of the technology as described.

## Claims

1. A composition for use in a method for inducing immune tolerance toward an antigen the composition comprising:
a) lipidic particles comprising the antigen and a phospholipid composition selected from the group consisting of:
i) phosphatidylcholine:phosphatidylinsositol (PC:PI) in a ratio of 40:60 to 60:40, and
ii) phosphatidyl serine:phosphatidylcholine (PS:PC) in a ratio of 10:90 to 30:70; and
wherein administration of the composition results in inducing immune tolerance in the individual to the antigen and wherein the antigen is a protein, polypeptide or peptide.

2. The composition of claim 1, wherein induction of immune tolerance is evidenced by one or more of the following:
i) reduction in antibody titer relative to the titer present prior to administration,
ii) increase in TGF-β and/or IL-10 levels
iii) reduction in one or more of the following: CD40, CD80, CD86, IL-6, IL-17.

3. The composition of claim 1, wherein the composition of i) further comprises cholesterol such that cholesterol is 1-20% of PC and PI together.

4. The composition of claim 3, wherein the cholesterol is 5-15% of PC and PI together.

5. The composition of claim 1, wherein the composition comprising PC and PI further comprises from 100 to 400 mM NaCl.

6. The composition of claim 5, wherein the composition further comprises from 0.1 to 1.0 mM calcium.

7. The composition of claim 1, wherein the ratio of PC:PI is 45:55 to 55:45.

8. The composition of claim 1, wherein the at least 50, 60, 70, 80 or 95% of the particles are from 60 to 140 nm.

9. The composition of claim 1, wherein the antigen associated with the lipidic particles is present in the ratio of 1:2,000 to 1:4,000.

10. A composition for use in a method for inducing immune tolerance comprising:
a) a lipidic composition comprising the antigen and O-phospho-L-serine (OPLS); and wherein the administration results in inducing immune tolerance in the individual to the antigen, wherein the antigen is a protein, polypeptide or a peptide.

11. The composition of claim 10, wherein induction of immune tolerance is evidenced by one or more of the following:
i) reduction in antibody titer relative to the titer present prior to administration,
ii) increase in TGF-β and/or IL-10 levels
iii) reduction in one or more of the following: CD40, CD80, CD86, IL-6, IL- 17.

12. A composition comprising stabilized lipidic nanoparticles comprising an antigen incoporated therein, wherein the phospholipids of the lipidic nanoparticles are selected from the group consisting of PC:PI ratio of 40:60 to 60:40 and wherein the lipid nanoparticles are stabilized by a buffer containing sodium chloride from 100 to 400 mM.

13. The composition of claim 12 further comprising 0.1 to 1.0 mM calcium.

14. The composition of claim 13, wherein the NaCl is from 150 to 300 mM.

15. The composition of claim 14, wherein the calcium is from 0.15 to 0.35 mM.
